# EUROPEAN PATENT APPLICATION

(11) **EP 4 800 703 A1**
(43) Date of publication of application: **02.09.2026**
(21) Application number: 26161441.6
(22) Date of filing: 27.02.2026
(51) Int. Cl.: G16H 30/40, G16H 50/30, G06T 7/00, G16H 50/20, G16H 50/70

(54) **HISTOGRAM-DERIVATIVE RISK CALIBRATION FOR ARTIFICIAL-INTELLIGENCE SCREENING SCORES**

(30) Priority: 28.02.2025 US 202563764573 P; 13.06.2025 US 202563823622 P; 31.07.2025 US 202563855290 P; 30.01.2026 US 202663972741 P; 30.01.2026 US 202663972737 P
(71) Applicant: Westerhoff, Malte, 12163 Berlin (DE); Visage Imaging GmbH, 12163 Berlin (DE)
(72) Inventor: Westerhoff, Malte, 12163 Berlin (DE)
(74) Representative: Schiweck Weinzierl Koch Patentanwälte Partnerschaft mbB

(57) **Abstract**

In an embodiment of the present invention, a method converts continuous scores from an AI disease classifier into clinically meaningful risk estimates. Historical score-outcome data are binned into percentiles, and the cumulative disease fraction is fitted with a differentiable curve for example a two-Beta mixture. The curve's derivative yields an instantaneous risk ratio that, combined with baseline prevalence, provides an absolute disease malady probability. Risk ratio and absolute probability ensures comparability across model versions, improving interpretability. The invention includes associated systems, software, and computer-readable media.

## Description

### FIELD OF INVENTION

The invention relates to computer-implemented medical-imaging diagnostics and, more particularly, to methods for transforming continuous output scores produced by Artificial-Intelligence (AI) classifiers for disease and malady detection into a clinically interpretable Instantaneous Risk Ratio and Absolute Risk in the disease or the malady. Further, the invention enables the Instantaneous Risk Ratio and Absolute Risk in the disease or the malady to remain stable across different AI classifier model versions.

### REFERENCE TO RELATED APPLICATIONS

This application claims priority to US provisional application no. 63/764,573, filed February 28, 2025, to US provisional applications no. 63/972,741, filed January 30, 2026, to US provisional applications no. 63/823622; filed June 13, 2025, to US provisional application no. 63/855,290, filed July 31, 2026 and to US provisional application no. 63/972,737, filed January 30, 2026. All references cited herein, including all patents and publications that are cited for any reason, including the U.S. provisional applications on which priority is based are incorporated by reference in their entirety and for all purposes.

### BACKGROUND OF THE INVENTION

Modem AI-based disease or malady detection image classification systems can be used in for example mammography to detect breast cancer, typically to automatically categorize images based on their content, such as identifying objects, scenes, or patterns. These machine learning algorithms typically use Convolutional Neural Networks (CNNs), Support Vector Machines (SVMs), or K-Nearest Neighbors (KNN) algorithms to extract features and thereby make predictions based on the features.

The advantages of an AI approach over traditional methods include that they can: (i) automate the process of image analysis, reducing human workload and time; (ii) be trained on large datasets, enabling them to learn complex patterns and achieve high accuracy; (iii) process vast amounts of image data quickly and efficiently, providing faster insights; and (iv) analyze images objectively, minimizing human bias.

AI-based detection image classification systems are used in medical imaging for tasks ranging from disease and malady detection to quality control; identifying the type of disease or malady, including tumors, aneurysms, arrhythmias, inflammation, and other abnormalities.

Osteoporosis is traditionally diagnosed by measuring BMD via DXA scans, but millions of CT scans taken for other reasons contain bone data that can be used to screen for osteoporosis opportunistically.

### SUMMARY OF THE INVENTION

In an embodiment of the present invention, a statistically principled method of mapping from a raw AI score is provided to determine the Instantaneous Risk Ratio (*RR(S)*)*.* In an embodiment of the present invention, the *RR(S)* can be used to determine the instantaneous disease or malady prevalence based on the neighborhood of score S relative to the cohort average. In an embodiment of the present invention, a statistically principled method of mapping from a raw AI score is provided to determine the Absolute Risk (*P*). In an embodiment of the present invention, in breast cancer, *P* the calibrated probability that a breast examined with a score *S* has cancer (i.e., C = 1) is given by Equation 1.

In an alternative embodiment of the present invention, in BMD, *P* the calibrated probability that a patient examined with a score *S* has osteoporosis (i.e., C = 1) is given by Equation 1.

In an embodiment of the present invention, *S* can be used to determine *P.*

### 1.1 Histogram Acquisition

In an embodiment of the present invention, the system collects paired data (S_i, C _i) from a validation cohort, according to Equation 2.$C _ i ∈ \left\{0,1\right\} ,$

Where the system indicates cancer confirmed by reliable ground truth and S_i is the corresponding score calculated by the AI classifier.

### 1.2 Percentile Mapping

In an embodiment of the present invention, the system converts each raw score S_i to an integer percentile rank, according to Equation 3.$t _ i = round \left(100⋅F_raw\left(S_i\right)\right) ,$
where *F_raw* is the empirical Cumulative Distribution Function (CDF) of scores in the same cohort. In an embodiment of the present invention, this percentile mapping (a rank-based quantile transform) decouples the calibration from any particular raw-score scale.

### 1.3 Empirical cumulative incidence curve N(t)

In various embodiments of the present invention, for each datapoint percentile *(t _i),* the Empirical cumulative incidence curve is given according to Equation 4.$N \left(t\right) = P \left(\left.C=1\right|T\leq t\right) = \left(g\leq t\right) / \left(total#cancers\right) ,$
where *g* = *# cancers within the percentile.*

In an embodiment of the present invention, *N(t)* is non-decreasing, ranges from 0 to 1, and represents the fraction of all detected cancers captured up to percentile *t.* In an alternative embodiment of the present invention, *N(t)* is monotonically increasing, ranges from 0 to 1, and represents the fraction of all detected cancers captured up to percentile *t.*

### 1.4 Smooth Parametric fit n(t)

In various embodiments of the present invention, the system can approximate *N(t)* with a differentiable cumulative-distribution function *n(t)* (e.g. a mixture of two Beta CDFs) given according to Equation 5.$n \left(t\right) = w ⋅ I _ x \left({α}_{1}, {β}_{1}\right) + \left(1-w\right) ⋅ I _ x \left({α}_{2}, {β}_{2}\right) ,$

### 1.5 Instantaneous Risk Ratio from Analytic derivative extraction

In various embodiments of the present invention, the Analytic derivative n'(t) obtained via the Beta Probability Density Function (PDF) yields the RR (Risk Ratio) (*RR(t)*) for cancer in percent, according to Equation 6.$RR \left(t\right) = 100 ⋅ n ′ \left(t\right) ,$

### 1.6 Absolute Risk

In various embodiments of the present invention, given a CBP (Cohort Baseline Prevalence) (*b*), the individual AR (Absolute Risk) (*P*) of cancer incidence is given by Equation 7.$P \left(\left.C=1\right|S=t\right) = RR \left(t\right) ⋅ b ,$
where *t* is the datapoint percentile.

In various embodiments of the present invention, **sections 1.1** through **1.6** can be important steps in generating the RR (*RR(S)*) and AR (*P*).

### 1.7 Curve Fitting Procedure

Implementation of the curve fitting procedure in python, for a percentile-normalized score data of a patient cohort is exemplified (where in the procedure shown in 1.7 *w, α₁, β₁, α₂,* and *β₂* are represented as w,a1,b1,a2, and b2).

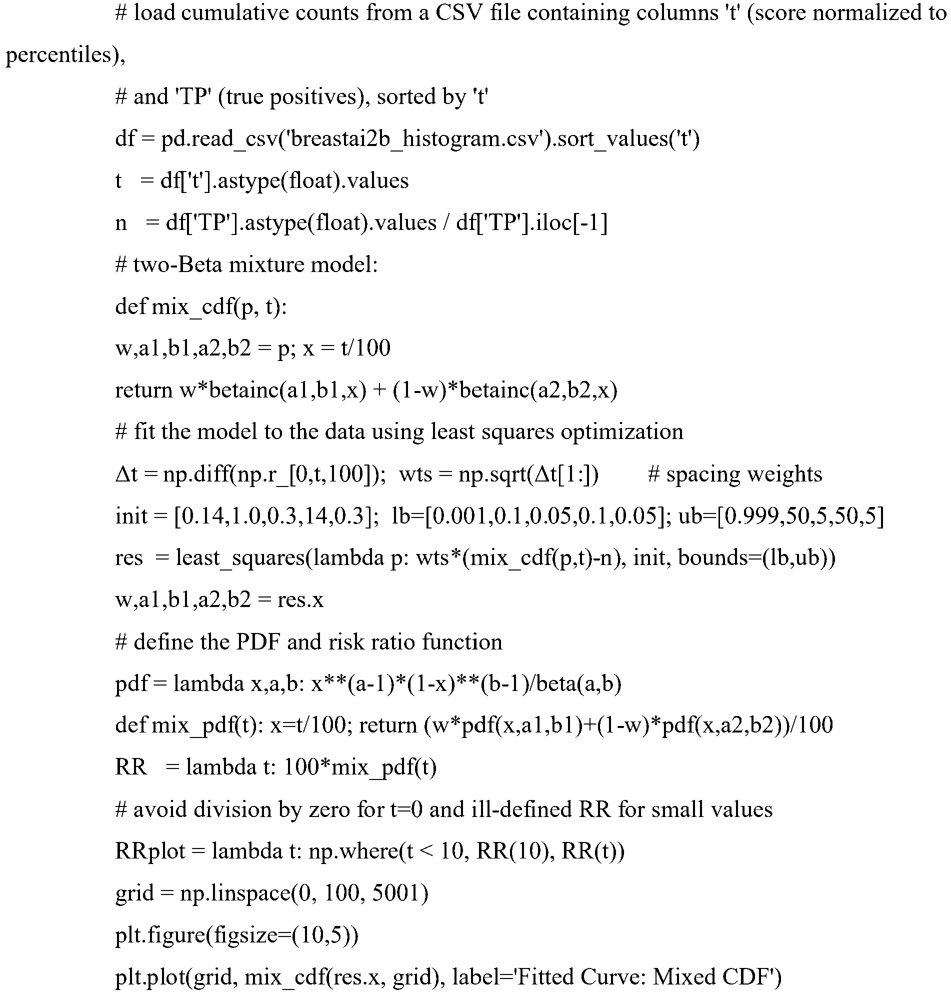

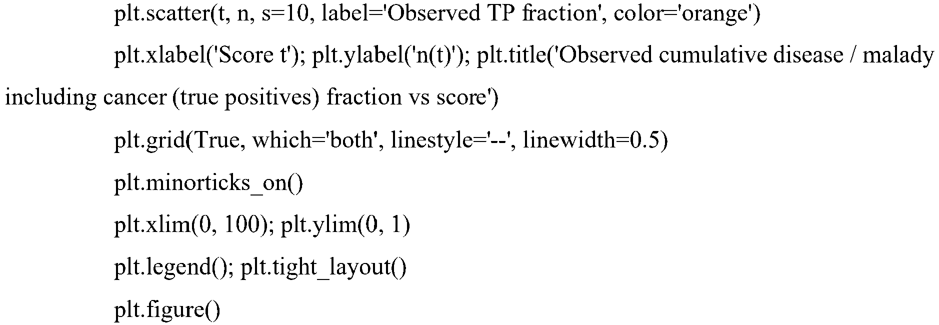

### 1.8 normalized Beta Cumulative Distribution Function

Definition of the Beta CDF *I_x(a,b).*

The normalized Beta cumulative-distribution function, *I_x(a,b)*, is defined as the ratio of the incomplete integral up to x to the complete Beta function according to Equation 8:
$I _ x \left(a, b\right) = B \left(x; a, b\right) / B \left(a, b\right) ,$

### Incomplete Beta function is given by Equation 9.

$B \left(x; a, b\right) = {\int }_{0}^{x} {t}^{a - 1} {\left(1-t\right)}^{b - 1} dt ,$
where 0 ≤ x ≤ 1, a > 0, and b > 0.

**Complete Beta function** is given by Equation 10,
$B \left(a, b\right) = {\int }_{0}^{1} {t}^{a - 1} {\left(1-t\right)}^{b - 1} dt = Γ \left(a\right) . Γ \left(b\right) / Γ \left(a+b\right) ,$
where Γ denotes the gamma function.

CBP of breast cancer is approximately 13% - approximately 15% of women. CBP of breast cancer varies widely with significant variations based on age, genetics, lifestyle, and demographics, often showing higher risk in older women and certain subgroups. For example, women with a sister with breast cancer, have a CBP of between approximately 25 - approximately 50%, where in this range approximately means plus or minus 10%. The CBP for breast cancer in women in the U.S. is 13%. The CBP for the recurrence of breast cancer in women in the U.S. is 13%. The CBP for the 5-year recurrence of breast cancer in women in the U.S. in which the breast cancer was detected at an early stage is 6%. The CBP for the 5-year recurrence of breast cancer in women in the U.S. in which advanced breast cancer (IIb - IIIc) was detected is 35%.

In an embodiment of the present invention, a method or system performs AI based assessment of breast cancer from CT / MRI scans. In an embodiment of the present invention, existing CT /MRI scans are analyzed and compared with historical CBP to identify an AR for the breast cancer, and accordingly the method adds no risk to the patient through additional exposure to radiation and no inconvenience, which improves patient acceptance.

### Generalized Equations 1A, 3A-10A.

In a separate embodiment of the present invention directed to determining RR, AR or IRR for a disease / malady, *P₁* the calibrated probability that a patient examined for a disease / malady with a score *S₁* has the disease / malady (i.e., C = 1) is also given by Equation 1A.${P}_{l} = \left(\left.C=l\right|{S}_{l}\right) ,$

In an embodiment of the present invention, the system collects paired data for a disease / malady (S*₁*_i, C_i) from a validation cohort, according to Equation 2.

Where the system indicates the disease / malady confirmed by reliable ground truth and S*₁*_i is the corresponding score calculated by the AI classifier, and each raw score S*₁*_i for the disease / malady can be converted to an integer percentile rank, according to Equation 3A.${t}_{1 _} i = round \left(100⋅F_raw\left({S}_{1 _}i\right)\right) ,$
where *F _raw* is the empirical Cumulative Distribution Function (CDF) of scores in the same cohort as known to one of ordinary skill in the art, where the percentile mapping decouples the calibration from any particular raw-score scale.

In various embodiments of the present invention, for each datapoint percentile *(t₁_i)*, the Empirical cumulative incidence curve is given according to Equation 4A. $N \left({t}_{1}\right) = {P}_{1} \left(C=1\left|T\leq {t}_{1}\right.\right) = \left({g}_{1}\leq {t}_{1}\right) / \left(\begin{matrix}total number patients with the disease / \\ malady\end{matrix}\right) ,$

Where *g₁* = the number of patients with the disease / malady within the percentile *t₁*, and *T* is the occurrence of the disease / malady which is less than or equal to the fraction of all detected disease / malady captured up to percentile *t₁*.

In an embodiment of the present invention, *N(t₁)* is non-decreasing, ranges from 0 to 1, and represents the fraction of all detected cases of the disease / malady captured up to percentile *t₁.* In an alternative embodiment of the present invention, *N(t₁)* is monotonically increasing, ranges from 0 to 1, and represents the fraction of all detected cases of the disease / malady captured up to percentile *t₁*.

In an embodiment of the present invention, the system can approximate *N(t₁)* with a differentiable cumulative-distribution function *n(t₁)* given according to Equation 5A. $n \left({t}_{1}\right) = w ⋅ I _ {x}_{1} \left({α}_{1}, {β}_{1}\right) + \left(1-w\right) ⋅ I _ {x}_{1} \left({α}_{2}, {β}_{2}\right) ,$

Where *x₁* = *t₁*/*100,* and *I_x₁(a,b)* is the normalized Beta cumulative-distribution function and *w*, *α₁, β₁, α₂,* and *β₂* are pre-computed parameters.

In an embodiment of the present invention, the Analytic derivative (*n'(t₁)*) obtained via the Beta Probability Density Function (PDF) yields the *IRR₁* (Instantaneous Risk Ratio for the disease / malady) (*RR(t₁)*) in percent, according to Equation 6A.$RR \left({t}_{1}\right) = 100 ⋅ n ′ \left({t}_{1}\right) ,$

In an embodiment of the present invention, given a CBP of a disease / malady, the individual AR (*P₁*) of a disease / malady incidence is given by Equation 7A.${P}_{1} \left(C=1\left|{S}_{1}={t}_{1}\right.\right) = RR \left({t}_{1}\right) ⋅ {b}_{1} ,$
where *t₁* is the datapoint percentile, and *b₁* is baseline prevalence for the disease / malady.

The normalized Beta cumulative-distribution function, *I_x₁(a,b)*, is defined as the ratio of the incomplete integral up to *x₁* to the complete Beta function according to Equation 8A.$I _ {x}_{1} \left(a, b\right) = B \left({x}_{1}; a; b\right) / B \left(a, b\right) ,$ $B \left(y\left(1\right);a,b\right) = {\int }_{0}^{y \left(1\right)} z {\left(1\right)}^{a - 1} {\left(1-z\left(1\right)\right)}^{b - 1} dz ,$ 0 ≤ *y(1)* ≤ 1, a > 0, and b > 0, and *x₁=y(1), and t₁ = z(1).*$B \left(a, b\right) = {\int }_{0}^{1} z {\left(1\right)}^{a - 1} {\left(1-z\left(1\right)\right)}^{b - 1} dz = Γ \left(a\right) . Γ \left(b\right) / Γ \left(a+b\right) ,$

### Osteoporosis Equations 1B, 3B-10B.

In a separate embodiment of the present invention directed to determining RR, AR or IRR for osteoporosis, *P₂* the calibrated probability that a patient examined with a score *S₂* has osteoporosis (i.e., C = 1) is given by Equation 1B.${P}_{2} \left(C=1\left|{S}_{2}\right.\right) ,$

In an embodiment of the present invention, the system collects paired data for osteoporosis (S*₂*_i, C_i) from a validation cohort, according to Equation 2.

A method of computing an osteoporotic fracture predictive risk score has been proposed, see Eldad Orna Bregman-Amital, U.S. Patent 10,588,589, and also a method of determining an osteoporosis risk score has been proposed, see M. Westehoff et al., U.S. Patent Application No.: 63/764,573 filed February 28, 2025, entitled "Method and System for Artificial Intelligence Based Bone Mineral Density Assessment from Computer Tomography Scans", which are herein incorporated by reference in their entirety.

Where the system indicates osteoporosis confirmed by reliable ground truth and S*₂*_i is the corresponding score calculated by the AI classifier, and each raw score S*₂*_i for trabecular attenuation in Hounsfield Units, generated from vertebrae in 3D CT images from routine 3DCT scans can be converted to an integer percentile rank, according to Equation 3B.${t}_{2 _} i = round \left(100⋅F_raw\left({S}_{2 _}i\right)\right) ,$
where *F_raw* is the empirical Cumulative Distribution Function (CDF) of scores in the same cohort, where the percentile mapping decouples the calibration from any particular raw-score scale.

In various embodiments of the present invention, for each datapoint percentile *(t_i)*, the Empirical cumulative incidence curve is given according to Equation 4B.$N \left({t}_{2}\right) = {P}_{2} \left(C=1\T\leq {t}_{2}\right) = \left({g}_{2}\leq {t}_{2}\right) / \left(total number patients with osteoporosis\right) ,$

Where *g₂* = number patients with osteoporosis within the percentile *t₂*, and *T* is the occurrence of osteoporosis which is less than or equal to the fraction of all detected osteoporosis captured up to percentile *t₂*.

In an embodiment of the present invention, *N (t₂)* is non-decreasing, ranges from 0 to 1, and represents the fraction of all detected cases of osteoporosis captured up to percentile *t₂*. In an alternative embodiment of the present invention, *N(t₂)* is monotonically increasing, ranges from 0 to 1, and represents the fraction of all detected cases of osteoporosis captured up to percentile *t₂*.

In an embodiment of the present invention, the system can approximate *N(t₂)* with a differentiable cumulative-distribution function *n(t₂)* given according to Equation 5B.$n \left({t}_{2}\right) = w ⋅ I _ {x}_{2} \left({α}_{1}, {β}_{1}\right) + \left(1-w\right) ⋅ I _ {x}_{2} \left({α}_{2}, {β}_{2}\right) ,$

Where *x₂ = t₂*/*100,* and *I_x₂ (a,b)* is the normalized Beta cumulative-distribution function.

In an embodiment of the present invention, the Analytic derivative n'(t*₂*) obtained via the Beta PDF yields the IRR*₂* (for osteoporosis) (*RR(t₂)*) in percent, according to Equation 6B.$RR \left({t}_{2}\right) = 100 ⋅ n ′ \left({t}_{2}\right) ,$

In an embodiment of the present invention, given a CBP of osteoporosis, the individual AR (*P₂*) of osteoporosis incidence is given by Equation 7B.${P}_{2} \left(C=1\left|{S}_{2}={t}_{2}\right.\right) = RR \left({t}_{2}\right) ⋅ {b}_{2} ,$
where *t₂* is the datapoint percentile, and *b₂* is baseline prevalence for osteoporosis.

The normalized Beta cumulative-distribution function, *I_x₂(a,b)*, is defined as the ratio of the incomplete integral up to *x₂* to the complete Beta function according to Equation 8B.$I _ {x}_{2} \left(a, b\right) = B \left({x}_{2}; a, b\right) / B \left(a, b\right) ,$$B \left(y\left(2\right); a, b\right) = {\int }_{0}^{y \left(2\right)} z {\left(2\right)}^{a - 1} {\left(1-z\left(2\right)\right)}^{b - 1} dz ,$

Where 0 ≤ *y(2)* ≤ 1, a > 0, and b > 0, and *x₂ = y(2), and t₂ = z(2).*$B \left(a, b\right) = {\int }_{0}^{1} z {\left(2\right)}^{a - 1} {\left(1-z\left(2\right)\right)}^{b - 1} dz = Γ \left(a\right) . Γ \left(b\right) / Γ \left(a+b\right) ,$

CBP of osteoporosis in U.S. adults aged 50 and over is approximately 4.5% in men and approximately 12.5% in women, where in this range approximately means plus or minus 10%. CBP of osteoporosis in adults 50-64 years is approximately 8.5% (approximately 3% men, approximately 13% women) and increases to approximately 17.5% in adults over 65 years (approximately 6% men, approximately 27% women). The prevalence of osteoporosis is highest in Mexican Americans (approximately 13%) and lowest in black Americans (approximately 4%).

In an embodiment of the present invention, a method or system performs AI based assessment of osteoporosis from CT / MRI scans. In an embodiment of the present invention, existing CT /MRI scans are analyzed and compared with historical CBP to identify an AR for osteoporosis, and accordingly the method adds no risk to the patient through additional exposure to radiation and no inconvenience, which improves patient acceptance.

### Pulmonary Embolism Equations 1C, 3C-10C.

In a separate embodiment of the present invention directed to determining RR, AR or IRR in a CTA of the lungs for Pulmonary Embolism (PE), *P₃* the calibrated probability that a patient examined with a score *S₃* has pulmonary embolism (i.e., C = 1) is given by Equation 1C.${P}_{3} \left(C=1\left|{S}_{3}\right.\right) ,$

In an embodiment of the present invention, the system collects paired data for PE (S*₃*_i, C_i) from a validation cohort, according to Equation 2.

A method of imaging the heart to generate raw scores has been proposed, see Andreas Fouras, U.S. Patent No. 9,576,354, issued February 21, 2017; a method of detecting pulmonary embolism and irregularities in vessels has also been proposed, see Andreas Fouras et al., U.S. Patent No. 11,278,256, issued March 22, 2022, a method of scanning vascular ill health and monitor blood vessels Chaminda Rajeev has also been proposed, Samarage et al., U.S. Patent No. 12,102,414, issued October 1, 2024; and a method of determining a PE risk score has also been proposed, see Edward Castillo et al., U.S. Patent Publication No. 20240296566, published September 5, 2024, entitled "Systems and Methods for Image Processing to Identify Patients with Pulmonary Embolism", which are each herein incorporated by reference in their entireties.

Where the system indicates PE confirmed by reliable ground truth and S*₃*_i is the corresponding score calculated by the AI classifier, and each raw score S*₃*_i for CT images from routine 3DCT scans can be converted to an integer percentile rank, according to Equation 3C.${t}_{3 _} i = round \left(100⋅F_raw\left({S}_{3 _}i\right)\right) ,$
where *F_raw* is the empirical CDF of scores in the same cohort as known to one of ordinary skill in the art, where the percentile mapping decouples the calibration from any particular raw-score scale.

In various embodiments of the present invention, for each datapoint percentile *(t₃_i),* the Empirical cumulative incidence curve is given according to Equation 4C.$N \left({t}_{3}\right) = {P}_{3} \left(C=1\left|T\leq {t}_{3}\right.\right) = \left({g}_{3}\leq {t}_{3}\right) / \left(total number patients with PE\right) ,$

Where *g₃* = number patients with PE within the percentile *t₃*, and *T* is the occurrence of PE which is less than or equal to the fraction of all detected PE captured up to percentile *t₃*.

In an embodiment of the present invention, *N(t₃)* is non-decreasing, ranges from 0 to 1, and represents the fraction of all detected cases of PE captured up to percentile *t₃*. In an alternative embodiment of the present invention, *N(t₃)* is monotonically increasing, ranges from 0 to 1, and represents the fraction of all detected cases of pulmonary embolisms captured up to percentile *t₃.*

In an embodiment of the present invention, the system can approximate *N(t₃)* with a differentiable cumulative-distribution function *n(t₃)* given according to Equation 5C.$n \left({t}_{3}\right) = w ⋅ I _ {x}_{3} \left({α}_{1}, {β}_{1}\right) + \left(1-w\right) ⋅ I _ {x}_{3} \left({α}_{2}, {β}_{2}\right) ,$

Where *x = t₃*/*100,* and *I_x₃(a,b)* is the normalized Beta cumulative-distribution function and *w*, *α₁, β₁, α₂,* and *β₂* are pre-computed parameters.

In an embodiment of the present invention, the Analytic derivative n'(t*₃*) obtained via the Beta PDF yields the IRR*₃* (for PE) (*RR(t₃)*) in percent, according to Equation 6C. $RR \left({t}_{3}\right) = 100 ⋅ n ′ \left({t}_{3}\right) ,$

In an embodiment of the present invention, given a CBP of PE, the individual AR (*P₃*) of pulmonary embolisms incidence is given by Equation 7C.${P}_{3} \left(\left.C=1\right|{S}_{3}=t\right) = {RR}_{3} \left(t\right) ⋅ {b}_{3} ,$

Where *t₃* is the datapoint percentile, and *b₃* is baseline prevalence for PE.

The normalized Beta cumulative-distribution function, *I_x₃(a,b)*, is defined as the ratio of the incomplete integral up to *x₃* to the complete Beta function according to Equation 8C.$I _ {x}_{3} \left(a, b\right) = B \left({x}_{3}; a, b\right) / B \left(a, b\right) ,$$B \left(y\left(3\right); a, b\right) = {\int }_{0}^{y \left(3\right)} z {\left(3\right)}^{a - 1} {\left(1-z\left(3\right)\right)}^{b - 1} dz ,$

Where 0 ≤ *y(3)* ≤ 1, a > 0, and b > 0, and *x₃ = y(3), and t₃ = z(3).*$B \left(a, b\right) = {\int }_{0}^{1} z {\left(3\right)}^{a - 1} {\left(1-z\left(3\right)\right)}^{b - 1} dz = Γ \left(a\right) . Γ \left(b\right) / Γ \left(a+b\right) ,$

CBP of PE is approximately 0.06% - approximately 0.12% in the U.S., where in this range approximately means plus or minus 10%. CBP of PE varies significantly with age (especially after 45) and risk factors (e.g., sex, obesity, cancer, surgery, immobility, genetic, and ethnicity), rising sharply in older adults, and showing increases during events like the COVID-19 pandemic where PE incidence increased by as much as 52%. Older individuals and certain sexes have higher risk.

In an embodiment of the present invention, a method or system performs AI based assessment of PE from CT / MRI scans. In an embodiment of the present invention, existing CT /MRI scans are analyzed and compared with historical CBP to identify an AR for PE, and accordingly the method adds no risk to the patient through additional exposure to radiation and no inconvenience, which improves patient acceptance.

### Brain Aneurysm Equations 1D, 3D-10D.

In a separate embodiment of the present invention directed to determining RR, AR or IRR for a Brain Aneurysm (BA), *P₄* the calibrated probability that a patient's CTA examined with a score *S₄* has a BA (i.e., C = 1) is given by Equation 1D.${P}_{4} \left(\left.C=1\right|{S}_{4}\right) ,$

In an embodiment of the present invention, the system collects paired data for BA (S*₄*_i, C_i) from a validation cohort, according to Equation 2.

A method of detecting a BA has been proposed, see Murray Reichner et al., U.S. Patent No. 10,607,341, issued March 31, 2020 which is herein incorporated by reference in its entirety.

Where the system indicates BA confirmed by reliable ground truth and S*₄*_i is the corresponding score calculated by the AI classifier, and each raw score S*₄*_i for CT images from routine 3DCT scans can be converted to an integer percentile rank, according to Equation 3D.${t}_{4} _ i = round \left(100⋅F_raw\left({S}_{4}_i\right)\right) ,$

where *F_raw* is the empirical CDF of scores in the same cohort, as known to one of ordinary skill in the art, where the percentile mapping decouples the calibration from any particular raw-score scale.

In various embodiments of the present invention, for each datapoint percentile *(t₄_i)*, the Empirical cumulative incidence curve is given according to Equation 4D.$N \left({t}_{4}\right) = {P}_{4} \left(\left.C=1\right|T\leq {t}_{4}\right) = \left({g}_{4}\leq {t}_{4}\right) / \left(total number patients with BA\right) ,$

Where *g₄* = number patients with BA within the percentile *t₄*, and *T* is the occurrence of the disease / malady which is less than or equal to the fraction of all detected disease / malady captured up to percentile *t₄*.

In an embodiment of the present invention, *N(t₄)* is non-decreasing, ranges from 0 to 1, and represents the fraction of all detected cases of brain aneurysms captured up to percentile *t₄.* In an alternative embodiment of the present invention, *N(t₄)* is monotonically increasing, ranges from 0 to 1, and represents the fraction of all detected cases of brain aneurysms captured up to percentile *t₄*.

In an embodiment of the present invention, the system can approximate *N(t₄)* with a differentiable cumulative-distribution function *n(t₄)* given according to Equation 5D.$n \left({t}_{4}\right) = w ⋅ I _ {x}_{4} \left({α}_{1}, {β}_{1}\right) + \left(1-w\right) ⋅ I _ {x}_{4} \left({α}_{2}, {β}_{2}\right) ,$

Where *x₄ = t₄*/*100,* and *I_x₄(a,b)* is the normalized Beta cumulative-distribution function and *w*, *α₁, β₁, α₂,* and *β₂* are pre-computed parameters.

In an embodiment of the present invention, the Analytic derivative n'(t*₄*) obtained via the Beta PDF yields the IRR*₄* (for BA) (*RR(t₄)*) in percent, according to Equation 6D.$RR \left({t}_{4}\right) = 100 ⋅ n ′ \left({t}_{4}\right) ,$

In an embodiment of the present invention, given a CBP, the individual AR (*P₄*) of BA incidence is given by Equation 7D. ${P}_{4} \left(C=1\left|{S}_{4}={t}_{4}\right.\right) = RR \left({t}_{4}\right) ⋅ {b}_{4} ,$

The normalized Beta cumulative-distribution function, *I_x₄(a,b)*, is defined as the ratio of the incomplete integral up to *x₄* to the complete Beta function according to Equation 8D.$I _ {x}_{4} \left(a, b\right) = B \left({x}_{4}; a, b\right) / B \left(a, b\right) ,$$B \left(y\left(4\right); a, b\right) = {\int }_{0}^{y \left(4\right)} z {\left(4\right)}^{a - 1} {\left(1-z\left(4\right)\right)}^{b - 1} dz ,$

Where 0 ≤ *y(4)* ≤ 1, a > 0, and b > 0, and *x₄ = y(4), and t₄ = z(4)*.$B \left(a, b\right) = {\int }_{0}^{1} z {\left(4\right)}^{a - 1} {\left(1-z\left(4\right)\right)}^{b - 1} dz = Γ \left(a\right) . Γ \left(b\right) / Γ \left(a+b\right) ,$

Where *t₄* is the datapoint percentile, and *b₄* is baseline prevalence for BA.

CBP of Brain Aneurysm (BA) is approximately 3% - approximately 5% in the U.S., where in this range approximately means plus or minus 10%. CBP of BA varies significantly with age (especially after 40, with peak prevalence between 35 - 60 years) and risk factors (e.g., sex, family history, and ethnicity), rising sharply in older adults.

The normalized Beta cumulative-distribution function, *I_x(a,b)*, is defined as the ratio of the incomplete integral up to x to the complete Beta function according to Equation 8.

In an embodiment of the present invention, a method or system performs AI based assessment of BA from CT / MRI scans. In an embodiment of the present invention, existing CT /MRI scans are analyzed and compared with historical CBP to identify an AR for BA, and accordingly the method adds no risk to the patient through additional exposure to radiation and no inconvenience, which improves patient acceptance.

### Peripheral Neuropathy Equations 1E, 3E-10E.

In a separate embodiment of the present invention directed to determining RR, AR or IRR for Peripheral Neuropathy (PN), *P₅* the calibrated probability that a MRI of a patient spine examined with a score *S₅* has PN (i.e., C = 1) is given by Equation 1E.${P}_{5} \left(C=1\left|{S}_{5}\right.\right) ,$

In an embodiment of the present invention, the system collects paired data for PN (S*₅*_i, C_i) from a validation cohort, according to Equation 2.

A method of predicting the risk of developing a neurological disorder based on a risk score derived in part from diagnostic imaging has been proposed, see Kenneth Rice, U.S. Patent Publication No. 20210180132, published June 17, 2021 which is herein incorporated by reference in its entirety.

Where the system indicates PN confirmed by reliable ground truth and S*₅*_i is the corresponding score calculated by the AI classifier, and each raw score S*₅*_i for CT images from routine 3DCT scans can be converted to an integer percentile rank, according to Equation 3E.${t}_{5} _ i = round \left(100⋅F_raw\left({S}_{5}_i\right)\right) ,$
where *F_raw* is the empirical CDF of scores in the same cohort, as known to one of ordinary skill in the art, where the percentile mapping decouples the calibration from any particular raw-score scale.

In various embodiments of the present invention, for each datapoint percentile *(t₅_i),* the Empirical cumulative incidence curve is given according to Equation 4E.$N \left({t}_{5}\right) = {P}_{5} \left(\left.C=1\right|T\leq {t}_{5}\right) = \left({g}_{5}\leq {t}_{5}\right) / \left(total number patients with PN\right) ,$

Where *g₅* = number patients with PN within the percentile *t₅*, and *T* is the occurrence of the disease / malady which is less than or equal to the fraction of all detected PN captured up to percentile *t₅.*

In an embodiment of the present invention, *N(t₅)* is non-decreasing, ranges from 0 to 1, and represents the fraction of all detected cases of peripheral neuropathy captured up to percentile *t₅.* In an alternative embodiment of the present invention, *N(t₅)* is monotonically increasing, ranges from 0 to 1, and represents the fraction of all detected cases of peripheral neuropathy captured up to percentile *t₅.*

In an embodiment of the present invention, the system can approximate *N(t₅)* with a differentiable cumulative-distribution function *n(t₅)* given according to Equation 5E.$n \left({t}_{5}\right) = w ⋅ I _ {x}_{5} \left({α}_{1}, {β}_{1}\right) + \left(1-w\right) ⋅ I _ {x}_{5} \left({α}_{2}, {β}_{2}\right) ,$

Where *x₅ = t₅*/*100,* and *I_x₅(a,b)* is the normalized Beta cumulative-distribution function and *w*, *α₁, β₁, α₂,* and *β₂* are pre-computed parameters.

In an embodiment of the present invention, the Analytic derivative n'(t*₅*) obtained via the Beta PDF yields the IRR*₅* (for PN) (*RR(t₅)*) in percent, according to Equation 6E.$RR \left({t}_{5}\right) = 100 ⋅ n ′ \left({t}_{5}\right) ,$

In an embodiment of the present invention, given a CBP, the individual AR (*P₅*) of PN incidence is given by Equation 7E.${P}_{5} \left(\left.C=1\right|{S}_{5}={t}_{5}\right) = RR \left({t}_{5}\right) ⋅ {b}_{5} ,$
where *t₅* is the datapoint percentile, and *b₅* is baseline prevalence for PN.

The normalized Beta cumulative-distribution function, *I_x₁(a,b),* is defined as the ratio of the incomplete integral up to *x₁* to the complete Beta function according to Equation 8A.$I _ {x}_{1} \left(a, b\right) = B \left({x}_{1}; a; b\right) / B \left(a, b\right) ,$$B \left(y\left(5\right);a,b\right) = {\int }_{0}^{y \left(5\right)} z {\left(5\right)}^{a - 1} {\left(1-z\left(5\right)\right)}^{b - 1} dz ,$

Where 0 ≤ *y(5)* ≤ 1, a > 0, and b > 0, and *x₅ = y(5), and t₅ = z(5).*$B \left(a, b\right) = {\int }_{0}^{1} z {\left(5\right)}^{a - 1} {\left(1-z\left(5\right)\right)}^{b - 1} dz = Γ \left(a\right) . Γ \left(b\right) / Γ \left(a+b\right) ,$

CBP of PN is approximately 1% - approximately 3% in the U.S. CBP of PN in adults over 40 is approximately 10% - approximately 14% and further increases with age (approximately 20% after age 70), where in this range approximately means plus or minus 10%. CBP of PN increases with risk factor such as diabetes.

In an embodiment of the present invention, a method or system performs AI based assessment of PN from CT / MRI scans. In an embodiment of the present invention, existing CT /MRI scans are analyzed and compared with historical CBP to identify an AR for PN, and accordingly the method adds no risk to the patient through additional exposure to radiation and no inconvenience, which improves patient acceptance.

### Inflammatory Bowel Disease Equations 1F, 3F-10F.

In a separate embodiment of the present invention directed to determining RR, AR or IRR for Inflammatory Bowel Disease (IBD), *P₆* the calibrated probability that a CT scan of the gut of a patient examined with a score *S₆* has IBD (i.e., C = 1) is given by Equation 1F.${P}_{6} \left(C=1\left|{S}_{6}\right.\right) ,$

In an embodiment of the present invention, the system collects paired data for IBD (S*₆*_i, C_i) from a validation cohort, according to Equation 2.

A method of determining a risk score of inflammatory bowel disease activity derived in part from diagnostic imaging has been proposed, see Jonathan Ng, U.S. Patent No. 12,394,524, published as US20220028547 January 27, 2022 which is herein incorporated by reference in its entirety.

Where the system indicates IBD confirmed by reliable ground truth and S*₆*_i is the corresponding score calculated by the AI classifier, and each raw score S*₆*_i for CT images from routine 3DCT scans can be converted to an integer percentile rank, according to Equation 3F.${t}_{6 _} i = round \left(100⋅F_raw\left({S}_{6 _}i\right)\right) ,$

Where *F_raw* is the empirical CDF of scores in the same cohort, where the percentile mapping decouples the calibration from any particular raw-score scale.

In various embodiments of the present invention, for each datapoint percentile *(t₆_i),* the Empirical cumulative incidence curve is given according to Equation 4F.$N \left({t}_{6}\right) = {P}_{6} \left(C=1\left|T\leq {t}_{6}\right.\right) = \left({g}_{6}\leq {t}_{6}\right) / \left(total number patients with IBD\right) ,$
where *g₆* = number patients with IBD within the percentile *t₆*, and *T* is the occurrence of the disease / malady which is less than or equal to the fraction of all detected disease / malady captured up to percentile *t₆.*

In an embodiment of the present invention, *N(t₆)* is non-decreasing, ranges from 0 to 1, and represents the fraction of all detected cases of IBD captured up to percentile *t₆.* In an alternative embodiment of the present invention, *N(t₆)* is monotonically increasing, ranges from 0 to 1, and represents the fraction of all detected cases of IBD captured up to percentile *t₆.*

In an embodiment of the present invention, the system can approximate *N(t₆)* with a differentiable cumulative-distribution function *n(t₆)* given according to Equation 5F.$n \left({t}_{6}\right) = w ⋅ I _ {x}_{6} \left({α}_{1}, {β}_{1}\right) + \left(1-w\right) ⋅ I _ {x}_{6} \left({α}_{2}, {β}_{2}\right) ,$
where *x₆ = t₆*/*100,* and *I_x₆ (a,b)* is the normalized Beta cumulative-distribution function and *w*, *α₁, β₁, α₂,* and *β₂* are pre-computed parameters.

In an embodiment of the present invention, the Analytic derivative n'(t*₆*) obtained via the Beta PDF yields the IRR*₆* (for IBD) (*RR(t₆)*) in percent, according to Equation 6F.$RR \left({t}_{6}\right) = 100 ⋅ n ′ \left({t}_{6}\right) ,$

In an embodiment of the present invention, given a CBP, the individual AR (*P₆*) of IBD incidence is given by Equation 7F.${P}_{6} \left(C=1\left|{S}_{6}={t}_{6}\right.\right) = {RR}_{6} \left(t\right) ⋅ {b}_{6} ,$
where *t₆* is the datapoint percentile, and *b₆* is baseline prevalence for IBD.

The normalized Beta cumulative-distribution function, *I_x₁(a,b)*, is defined as the ratio of the incomplete integral up to *x₁* to the complete Beta function according to Equation 8F.$I _ {x}_{6} \left(a, b\right) = B \left({x}_{6}; a; b\right) / B \left(a, b\right) ,$$B \left(y\left(6\right):a,b\right) = {\int }_{0}^{y \left(6\right)} z {\left(6\right)}^{a - 1} {\left(1-z\left(6\right)\right)}^{b - 1} dz ,$

Where 0 ≤ *y(6)* ≤ 1, a > 0, and b > 0, and *x₆ = y(6), and t₆ = z(6)*.$B \left(a, b\right) = {\int }_{0}^{1} z {\left(6\right)}^{a - 1} {\left(1-z\left(6\right)\right)}^{b - 1} dz = Γ \left(a\right) . Γ \left(b\right) / Γ \left(a+b\right) ,$

CBP of IBD is approximately 0.9% in the U.S. CBP of IBD shows a prevalence is white individuals (1.2%) compared with 0.3% for black Americans.

CBP of Crohn's disease is approximately 0.3% - approximately 0.6% in the U.S. CBP of Crohn's disease shows prevalence is also higher in white individuals and peaks in early adulthood, with incidence rates increasing in the U.S.

In an embodiment of the present invention, a method or system performs AI based assessment of IBD from CT / MRI scans. In an alternative embodiment of the present invention, a method or system performs AI based assessment of IBD from CT / MRI scans. In an embodiment of the present invention, existing CT /MRI scans are analyzed and compared with historical CBP to identify an AR for IBD. In an embodiment of the present invention, existing CT /MRI scans are analyzed and compared with historical CBP to identify an AR for Crohn's disease, and accordingly the method adds no risk to the patient through additional exposure to radiation and no inconvenience, which improves patient acceptance.

### Celiac Disease Equations 1G, 3G-10G.

In a separate embodiment of the present invention directed to determining RR, AR or IRR for Celiac Disease (CD), *P₇* the calibrated probability that a MRE scan of the bowel of a patient with a score *S₇* has CD (i.e., C = 1) is given by Equation 1B.${P}_{7} \left(C=1\left|{S}_{7}\right.\right) ,$

In an embodiment of the present invention, the system collects paired data for CD (S*₇*_i, C_i) from a validation cohort, according to Equation 2.

Where the system indicates Celiac disease confirmed by reliable ground truth and S*₇*_i is the corresponding score calculated by the AI classifier, and each raw score S*₇*_i for CT images from routine 3DCT scans can be converted to an integer percentile rank, according to Equation 3G.${t}_{7} _ i = round \left(100⋅F_raw\left({S}_{7}_i\right)\right) ,$
where *F_raw* is the empirical CDF of scores in the same cohort, where the percentile mapping decouples the calibration from any particular raw-score scale.

In various embodiments of the present invention, for each datapoint percentile *(t₇_i),* the Empirical cumulative incidence curve is given according to Equation 4G.$N \left({t}_{7}\right) = {P}_{7} \left(C=1\left|T\leq {t}_{7}\right.\right) = \left({g}_{7}\leq {t}_{7}\right) / \left(total number patients with CD\right) ,$

Where *g₇* = number patients with Celiac disease within the percentile *t₇*, and *T* is the occurrence of the disease / malady which is less than or equal to the fraction of all detected disease / malady captured up to percentile *t₇.*

In an embodiment of the present invention, *N(t₇)* is non-decreasing, ranges from 0 to 1, and represents the fraction of all detected cases of Celiac disease captured up to percentile *t₇.* In an alternative embodiment of the present invention, *N(t₇)* is monotonically increasing, ranges from 0 to 1, and represents the fraction of all detected cases of Celiac disease captured up to percentile *t₇.*

In an embodiment of the present invention, the system can approximate *N(t₇)* with a differentiable cumulative-distribution function *n₇(t)* given according to Equation 5G.$n \left({t}_{7}\right) = w ⋅ I _ {x}_{7} \left({α}_{1}, {β}_{1}\right) + \left(1-w\right) ⋅ I _ {x}_{7} \left({α}_{2}, {β}_{2}\right) ,$
where *x₇* = *t₇*/*100,* and *I_x(a,b)* is the normalized Beta cumulative-distribution function and *w*, *α₁, β₁, α₂,* and *β₂* are pre-computed parameters.

In an embodiment of the present invention, the Analytic derivative n'(t*₇*) obtained via the Beta PDF yields the IRR*₇* (for CD) (*RR(t₇)*) in percent, according to Equation 6G.$RR \left({t}_{7}\right) = 100 ⋅ n ′ \left({t}_{7}\right) ,$

In an embodiment of the present invention, given a CBP, the individual AR (*P₇*) of CD incidence is given by Equation 7G.${P}_{7} \left(C=1\left|{S}_{7}={t}_{7}\right.\right) = RR \left({t}_{7}\right) ⋅ {b}_{7} ,$

Where *t₇* is the datapoint percentile, and *b₇* is baseline prevalence for CD.

The normalized Beta cumulative-distribution function, *I_x₇(a,b),* is defined as the ratio of the incomplete integral up to *x₇* to the complete Beta function according to Equation 8G.$I _ {x}_{7} \left(a, b\right) = B \left({x}_{7}; a, b\right) / B \left(a, b\right) ,$$B \left(y\left(7\right); a, b\right) = {\int }_{0}^{y \left(7\right)} z {\left(7\right)}^{a - 1} {\left(1-z\left(7\right)\right)}^{b - 1} dz ,$

Where 0 ≤ *y(7)* ≤ 1, a > 0, and b > 0, and *x₇ = y(7), and t₇* = *z(7).*$B \left(a, b\right) = {\int }_{0}^{1} z {\left(7\right)}^{a - 1} {\left(1-z\left(7\right)\right)}^{b - 1} dz = Γ \left(a\right) . Γ \left(b\right) / Γ \left(a+b\right) ,$

CBP of Celiac disease is approximately 0.4% - approximately 1.4% in the U.S., where in this range approximately means plus or minus 10%. CBP of Celiac disease shows prevalence is higher in white individuals. Further, the incidence and prevalence of Celiac disease is increasing in the U.S.

The normalized Beta cumulative-distribution function, *I_x(a,b)*, is defined as the ratio of the incomplete integral up to x to the complete Beta function according to Equation 8.

In an embodiment of the present invention, a method or system performs AI based assessment of Celiac disease from CT / MRI scans. In an embodiment of the present invention, existing CT /MRI scans are analyzed and compared with historical CBP of Celiac disease to identify an AR for Celiac disease, and accordingly the method adds no risk to the patient through additional exposure to radiation and no inconvenience, which improves patient acceptance.

These and other aspects of the invention are evident in the drawings and in the description that follows.

### BRIEF DESCRIPTION OF THE DRAWINGS

This invention is described with respect to specific embodiments thereof. Additional features can be appreciated from the Figures in which:
**FIG. 1** depicts a flow chart showing a schematic representation of the method of generating the RR and the AR of breast cancer from a Volumetric Digital Breast Tomosynthesis Image of a patient, according to an embodiment of the invention;
**FIG. 2** shows (i) the counts of true-positive outcome *N(t)* in ground-truth versus percentile-calibrated Score t (circle) and (ii) a fitted continuous and differentiable mixed-beta function (continuous line) curve, according to an embodiment of the invention; and
**FIG. 3** shows the Risk Ratio plotted on a logarithmic axis versus the Score t calculated according to an embodiment of the invention.
**FIG. 4A** depicts a flow chart showing a schematic representation of a method to determine bone mineral density status of a segmented vertebrae, according to an embodiment of the invention.
**FIG. 4B** depicts a flow chart showing a schematic representation of the method depicted in **FIG. 4A****,** where the segmented vertebrae are labelled, according to an embodiment of the invention.
**FIG. 4C** depicts a flow chart showing a schematic representation of the method depicted in **FIG. 4A****,** where each segmented vertebra is checked to determine that they form a single connected vertebra, according to an embodiment of the invention.
**FIG. 4D** depicts a flow chart showing a schematic representation of the method depicted in **FIG. 4A****,** where each segmented vertebra is checked to determine that they are in the correct anatomical sequence, according to an embodiment of the invention.
**FIG. 4E** depicts a flow chart showing a schematic representation of the method depicted in **FIG. 4D****,** where consistency checks are performed on the one or more segmented vertebrae to ensure valid identification of the one or more segmented vertebrae, according to an embodiment of the invention.
**FIG. 4F** depicts a flow chart showing a schematic representation of the method depicted in **FIG. 4A****,** where the volume of the three-dimensional spherical Region of Interest is a predetermined percentage of the volume of the one or more segmented vertebrae, according to an embodiment of the invention.
**FIG. 4G** depicts a flow chart showing a schematic representation of the method depicted in **FIG. 4A****,** where the position of the spherical ROI avoids the posterior central region of the one or more segmented vertebrae where a basivertebral foramen is located, according to an embodiment of the invention.
**FIG. 4H** depicts a flow chart showing a schematic representation of the method depicted in **FIG. 4A****,** where the position targets a region of relatively low attenuation within the one or more segmented vertebrae, according to an embodiment of the invention.
**FIG. 4J** depicts a flow chart showing a schematic representation of the method depicted in **FIG. 4A****,** where the plurality of voxel intensities is in Hounsfield Units, according to an embodiment of the invention.
**FIG. 4K** depicts a flow chart showing a schematic representation of the method depicted in **FIG. 4A****,** where representative attenuation values are calculated by computing a median of the plurality of voxel intensities, according to an embodiment of the invention.
**FIG. 4L** depicts a flow chart showing a schematic representation of the method depicted in **FIG. 4A****,** where the acquisition parameters are the model of the scanner or the X-ray tube voltage used to generate the 3DCTI, according to an embodiment of the invention.
**FIG. 4M** depicts a flow chart showing a schematic representation of the method depicted in **FIG. 4A****,** where the calibrated attenuation values are compared with a population-based reference data stratified by one or both age and sex to determine a percentile ranking, according to an embodiment of the invention.
**FIG. 4N** depicts a flow chart showing a schematic representation of the method depicted in **FIG. 4A****,** where the calibrated attenuation values are compared with a population-based reference data stratified by one or both age and sex to determine a diagnostic category, according to an embodiment of the invention.

### DEFINITIONS

The transitional term 'comprising' is synonymous with 'including", 'containing", or 'characterized by", is inclusive or open-ended and does not exclude additional, unrecited elements or method steps.

The transitional phrase 'consisting of' excludes any element, step, or ingredient not specified in the claim, but does not exclude additional components or steps that are unrelated to the invention such as impurities ordinarily associated with a composition.

The transitional phrase 'consisting essentially of' limits the scope of a claim to the specified materials or steps and those that do not materially affect the basic and novel characteristic(s) of the claimed invention.

The term 'Study' refers in the usual and customary sense to the set of images produced by an examination. A Study consists of one or more images. The images can be grouped into one or more image series. Each image, each series, and the whole Study can have different parameters attached. For medical images these can be defined by the Digital Imaging and Communication in Medicine (DICOM) standard.

Some or all of the images in a Study can form one or more three-Dimensional 'Volumes.' For 3D CT modalities, often each individual image in the series corresponds to a volume, but that is not a requirement. For example, a cardiac CT may contain multiple 3D volumes covering the heart, each corresponding to a different point in the cardiac cycle, and all of the images belonging to all of these volumes being grouped into the same series.

The term 'Hanging Protocol' refers in the usual and customary sense to specific conventions how X-Ray films are arranged at a light box.

The term 'Display Protocol' refers in the usual and customary sense to the way images are displayed in a computer system, specifically the selection of the images to be displayed, the layout of the images, as well as the rendering parameters and styles.

The term 'View' refers in the usual and customary sense to data corresponding to a digital image view of a Set of Images rendered with a given set of rendering parameters and rendering modes.

The term 'Viewport' refers in the usual and customary sense to the logical part of the screen on the client computer in which a particular View is displayed, for example the user interface on the client computer can contain four rectangular Viewports of which three show a frontal, left, and bottom view respectively of a particular data, while the fourth viewer might show a 2D cross section through the same or a different data set.

The term 'Sets of Images' or 'Image Set' refers in the usual and customary sense to one or more images, selected based on the rules.

The term 'Study Selection Rules' refers in the usual and customary sense to the rules used to select the studies to be displayed.

The term 'Protocol Selection Rules' refers in the usual and customary sense to the rules used to select the layout of the images to be displayed.

The term 'Image Set Rules' refers in the usual and customary sense to the rules used to form Image Sets from the images of one or more Study by applying selection, sorting, and breaking rules.

The term 'Style Rules' refers in the usual and customary sense to the rules to determine which rendering type, rendering style, and rendering parameters are used for a particular Image Set in a particular viewer.

The terms "Volume Rendering" (verb) and "Rendered Volume" (RV) (noun) refer in the usual and customary sense to computer graphics visualization techniques that create e.g., a 2D and / or a 3D image from 3D image data sets, where a typical 3D image data set is a large number of 2D slice images acquired by a CT scanner and stored in a data structure. A typical brain MRI slice or breast CTI slice can comprise between 65,536 and 262,144 pixels. As known by a person of ordinary skill in the art, a large 2D data set of an organ (e.g. a brain, or a breast [a specialized glandular organ]) can be used to generate a 3D RV of the organ. In a typical breast CTI, 700-1200 images (of 1 mm thick slices, the actual number of images required depends on the breast size) can be required to create a full breast 3D VR. In a typical brain MRI, 300-400 2D images (of 1 - 3 mm thick slices, from the vertex to the foramen magnum) can be required to create a full brain 3D VR. VR techniques that generate a RV include shaded volume, maximum intensity projection (MIP), oblique slicing or multi-planar reformats (MPR), axial/sagittal and coronal slice display, and thick slices (also called slabs).

The term 'anatomical characteristic' includes the group consisting of one or more of spine, chest, abdomen, breast, shoulder, trapezius, arm, elbow, wrist, finger, pelvis, hip, fibula, knee, tibula, ankle, foot, neck, head, temporomandibular junction, face, brain, dentition, sinus, adrenals, retina, pituitary, and prostate. The anatomical characteristic can include the Body Part Examined. An anatomical characteristic can be either natural or pathologic. A natural anatomical characteristic of a patient would be the presence of seven cervical vertebrae. A pathologic anatomical characteristic of a patient would be the presence of only six cervical vertebrae.

The term 'anatomical feature' refers to a medical condition, e.g., whether a fracture is present in a given image or volume or whether a bleeding is present in a given image or volume. An anatomical feature can be a fractured fibula, a herniated disc, urethral bleeding, e.g. bleeding with benign prostate hyperplasia, lacerated breast, gunshot wound to the chest, infection by *Treponema pertenue* giving rise to YAWS lesion in left distal leg. An anatomical feature is pathologic. In an embodiment of the invention, if ParameterA is fibula then the anatomical feature can be a fractured fibula.

The term 'disease-based characteristic' can be selected from the type of disorder or disease being evaluated, e.g., a diagnosis of lung cancer. The disease-based characteristic can include the Body Part Examined. A disease-based characteristic can be pathologic.

The phrase 'vertebral body' refers to the main, weight-bearing section of a single vertebra. The phrase 'one or more vertebrae' refers to one vertebra or more vertebrae, meaning from one to the entire set of spinal bones in the vertebral column. The phrase 'one or more segmented vertebrae' or 'segmented vertebra' refers to each separate vertebra in the vertebral column.

The phrase 'Convolutional Neural Networks' or CNN is a feedforward neural network that learns features through kernel optimization and which can be used to precisely identify organs, bones, and other bodies in 3D images.

A render server program is described in United States Patent No. 9,904,969, entitled 'Multi-User Mult-GPU Render Server Apparatus and Methods', inventors M. Westerhoff et al., which issued February 27, 2018, and which is herein expressly incorporated by reference in its entirety and for all purposes. A rule based render server program is described in United States Patent No. 8,976,190, entitled 'Method and System for Rule Based Display of Sets of Images', inventors M. Westerhoff et al., which issued March 10, 2015, and which is herein expressly incorporated by reference in its entirety and for all purposes. A program for visualizing DBT is described in United States Patent No. 9,984,478, entitled 'Apparatus and Method for Visualizing Digital Breast Tomosynthesis and Other Volumetric Images', inventors M. Westerhoff et al., which issued May 29, 2018, and which is herein expressly incorporated by reference in its entirety and for all purposes. A program using Convolutional Neural Network analysis of image content based parameters is described in United States Patent No. 10,909,679, entitled 'Method and System For Rule-Based Display of Sets of Images Using Image Content Derived Parameters', inventors M. Westerhoff et al., which issued October 1, 2020, and which is herein expressly incorporated by reference in its entirety and for all purposes. A program for transfer of large data sets is described in United States Patent No. 9,509,802, entitled 'Method and System for Transferring Data to Improve Responsiveness when Sending Large Data Sets', inventors D Stalling et al., which issued November 29, 2016, and which is herein expressly incorporated by reference in its entirety and for all purposes. A program for anonymized data transfer is described in United States Patent No. 11,599,672, entitled 'Method and System for Anonymized Display and Data Export', inventors D Stalling et al., which issued March 7, 2023, and which is herein expressly incorporated by reference in its entirety and for all purposes. An AI based program for determining bone density is described in U.S. Provisional Patent Application 63/764,573 'A Method and System for AI Based Bone Mineral Density Assessment from CT Scans' inventors M. Westerhoff et al., which was filed February 28, 2025, and is herein expressly incorporated by reference in its entirety and for all purposes.

The phrase 'basivertebral foramen' (BF) means a bone opening in the back wall of the vertebrae that contains the basivertebral veins and nerves. The BF is present in every vertebra of the spine.

The word 'osteopenia' is a condition characterized by mild to moderate bone loss resulting in a lowered BMD. Osteopenia makes bones weaker and more susceptible to fractures. A T-score from a DXA scan above -1.0 can indicate a patient has a normal BMD. A T-score from a DXA scan between -1.0 to -2.5 can indicate mild to moderate bone loss and can result in a diagnosis of osteopenia.

The word 'osteoporosis' is a condition characterized by severe bone loss resulting in a low BMD. Osteopenia makes bones weak, porous, and brittle and susceptible to higher risk of fractures from activities such as minor falls, bending, or even coughing. Fractures to the hip, spine, and wrist are common. A T-score from a DXA scan below -2.5 can indicate low BMD and can result in a diagnosis of osteoporosis.

The phrase 'osteopenic trabecular region' means a part of bone that is affected by osteopenia. For example, the osteopenic travecular region is that part of the bone that thins due to osteopenia.

The phrase 'vertebral trabecular bone region' means a part of bone that is inspected to determine if the patient is affected by osteopenia.

The phrase "spherical Regions of Interest" or "spherical ROI" means a 3D spherical volume for measuring bone tissue density within a specific area to extract quantitative data. By using a volumetric ROI (covering many voxels in 3D) instead of e.g., a single 2D slice patch, the influence of local inhomogeneities can be reduced and a more reliable measure of overall trabecular density can be obtained. The spherical ROI's center is chosen such that: (i) the sphere lies completely inside the vertebral body segmentation, (ii) the median HU value within the sphere is the lowest obtainable while satisfying that the sphere lies completely inside the vertebral body segmentation, and (iii) the sphere is located anterior to the basivertebral foramen (thus excluding the posterior vascular channel region).

The 'median HU value' is found by ranking the HU values and picking the middle one, if there is an even number of voxels, or an average of the two central values if there is an odd number of voxels.

The "representative attenuation value for a vertebra" can be computed once the spherical ROI is determined as the average HU value of all voxels inside the spherical ROI. In an embodiment of the invention, the average HU value is the median HU value of all voxels inside the spherical ROI. The "representative attenuation value for the one or more spherical ROI" is the average HU value of all voxels inside the spherical ROI. The average HU value for each vertebra can be calibrated to a standardized scale to generate the 'calibrated attenuation values'.

The "population-based reference data" refers in the usual and customary sense to a representative sample of a population with health norms that is used to assess and inform policy on osteopenia and osteoporosis.

The phrase "bone mineral density" (BMD) refers in the usual and customary sense to a measure of the calcium and minerals in bones, indicating their fracture risk, with lower density BMD making bones brittle.

The phrase 'basivertebral veins' means the large, tortuous veins of the trabecular bone of vertebral bodies that drain into the internal and external vertebral venous plexuses. They emerge from the vertebral bodies horizontally through foramina in the bone. Anteriorly, they drain into the external vertebral venous plexuses and posteriorly, they converge into the basivertebral canal and drain into the anterior internal vertebral venous plexus.

The phrase 'basivertebral venous plexus' refers to a network of interconnected longitudinal channels located beneath the bony structures within the basivertebral canal. The plexus lacks a valve and relies on posture and respiration for drainage.

The hip joint is a ball-and-socket spheroidal synovial joint. The ball is the femoral head, and the socket is the acetabulum. The hip joint is the articulation of the pelvis with the femur, which connects the patient's axial skeleton with the lower extremity.

The phrase 'femoral neck' refers to the part of the thigh bone that connects the femoral head to the femoral shaft. The femoral neck is a flattened, pyramidal process of bone which is roughly cylindrical and flares out as it joins the shaft, it is located just below the ball of the hip joint.

The shoulder joint is a ball-and-socket spheroidal synovial joint. The shoulder joint is made up of the shoulder blade (scapula), the collar bone (clavicle) and upper arm (humerus). The acromioclavicular joint joins the top of your shoulder blade (acromion) and your collarbone together. The glenohumeral joint joins the rounded top of your upper arm bone into your shoulder blade (glenoid cavity of the scapula). The shoulder joint is formed by the articulation between the head of the humerus and the glenoid cavity of the scapula. It is the most mobile joint in the body.

The phrase 'humeral neck' refers to constricted part of the humerus bone that connects the head of the humerus to the body of the bone. It is located below the head of the humerus.

The knee joint is a ball-and-socket spheroidal synovial joint. The knee joint is made up of the thigh bones (femur and fibula), shin bone (tibia), and the knee cap (patella). The tibiofemoral joint is a modified hinge synovial joint between the distal femur and the proximal tibia. The tibiofemoral joint is the weight bearing joint of the knee. The patella lies in an indentation of the femur known as the intercondylar groove. The patellofemoral joint is between the patella and the femur.

The phrase 'distal femur' refers to the lower part of the thigh bone, just above the knee joint. It's shaped like a trapezoid and flares out like an upside-down funnel.

The axial skeleton is the central core of the human skeleton, consisting of the bones that form the vertical axis of the body. The axial skeleton includes bones in your head, neck, back, pelvis, and chest. In contrast, the femur, the scapula, the clavicle and the humerus are all part of the appendicular skeleton.

The term 'anatomical region' refers to an area of a body. That is the anatomical region can include the head, the spine, the hip, the shoulder, and the knee. When the anatomical region is the spine, then the anatomical bones in the anatomical region are the one or more vertebrae. When the anatomical region is the hip, then the anatomical bones in the anatomical region are the one or more bones in the one or both hip joints. When the anatomical region is the shoulder, then the anatomical bones in the anatomical region are the one or more bones in the one or both shoulder joints. When the anatomical region is the knee, then the anatomical bones in the anatomical region are the one or more bones in the one or both knee joints.

The terms 'True positive outcome' and '*N(t)*', refer in the usual and customary sense to 'true' or 'positive' outcomes in data, see Equations 4A or 4B. In an embodiment of the present invention, the data sets can be historical.

The term 'Cohort Baseline Prevalence', 'CBP', 'prevalence', '*b*', refer in the usual and customary sense to the existing rate or proportion of a condition, disease, malady, or characteristic (e.g., proportion of positive cases) within a cohort, see Equation 7. In an embodiment of the invention, the product of RR and the specific disease or malady CBP is used to calculate AR for the specific disease or malady.

The phrase 'Artificial Information Classifier' or AIC refers in the usual and customary sense to a machine learning algorithm that organizes data into predefined categories or classes based on its characteristic. In an embodiment of the invention, the AIC generates a histogram.

The term 'prevalence of the classifier' refers in the usual and customary sense to the proportion of positive cases within a population being classified.

The terms 'Absolute Risk', 'AR', '*P*', refer in the usual and customary sense to the calibrated probability that an organ or body part examined with score *S* has a specified disease or malady. The AR for cancer refers to the calibrated probability that a patient's organ or body part examined with score *S* has a specified cancer. The AR for breast cancer refers to the calibrated probability that a patient's breast examined with score *S* has breast cancer. The AR for a heart arrythmia refers to the calibrated probability that a patient's heart examined with score *S* has an arrythmia. The AR for a brain aneurysm refers to the calibrated probability that a patient's brain examined with score *S* has an aneurysm. The AR for peripheral neuropathy refers to the calibrated probability that a patient's sensory nerves examined with score *S* indicates the patient has a peripheral neuropathy. The AR for peripheral neuropathy refers to the calibrated probability that a patient's motor nerves examined with score *S* indicates the patient has a peripheral neuropathy. The AR for peripheral neuropathy refers to the calibrated probability that a patient's autonomic nerves examined with score *S* indicates the patient has a peripheral neuropathy. The AR for IBD refers to the calibrated probability that a patient's stomach with a gastrointestinal examination score *S* has IBD. The AR for Celiac disease refers to the calibrated probability that a patient's stomach with a gastrointestinal examination score *S* has Celiac disease. The AR for osteoporosis refers to the calibrated probability that a patient's trabecular bone vertebrae examination score *S* has osteoporosis.

Gluten proteins comprise a large family of storage proteins found in cereal grain seeds, where 'gluten' is the water insoluble seed storage protein found in the Triticeae grains that includes wheat, barley and rye. Gluten is thought to trigger an immune response in a person's small intestine. Over time, this reaction produces inflammation that damages the small intestinal lining and causes mal-absorption preventing absorption of some important nutrients by the person. There is a genetic basis for Celiac disease as people who have a first degree relative with Celiac disease have a 1 in 22 chance of developing Celiac disease themselves. It is estimated that Celiac disease occurs in more than 70 million people worldwide. There is no cure for Celiac disease, no representative animal model from which to study the immune response mechanisms, and little hope of completely changing the immunogenic nature of all Triticeae grains through bioengineering. In order to ameliorate the effects of Celiac disease, people affected can try and have a 'gluten free' diet. Currently, a blood test and a biopsy are required in order to positively diagnose that a person has Celiac disease. However, MRI can also be used in diagnosing Celiac disease, particularly when looking for specific intestinal and extra-intestinal abnormalities.

In an embodiment of the invention, an Artifact Elimination Test (AET) can be used to generate a score. First a 3D volume of an organ is generated from CT scans (or an MRI). The AET is then used to determine if there is a region of tissue in an organ that is (i) at the border of a change in radiopacity and (ii) that remains fixed in space irrespective of the viewing angle. In an embodiment of the invention, the position of a feature identified in the 3D volume that moves as the viewing position moves can be eliminated as an artifact and is thus 'Scored' as S= 0. In an embodiment of the invention, the position of a feature identified in the 3D volume that remains constant cannot be eliminated as an artifact and is thus 'Scored' as S = 1.

The phrase 'a plurality of scores' means in the usual and customary sense a numerical value produced by a model or measurement system that can take on an infinite number of values within a given range, rather than being restricted to distinct, separate categories, i.e. a plurality of scores means a plurality of continuous output scores. In contrast, a number of 'score bins' can be generated by binning the continuous output score into discrete categorical bins.

The terms 'Instantaneous Risk Ratio' and 'IRR' refer in the usual and customary sense to a statistical measure used in research to compare the instantaneous rate or occurrence, or death, between two groups at a specific point in time, rather than looking at the total cumulative risk over a long period. By multiplying the analytic derivative (*n(t)*) by a scaling factor corresponding to the number of score bins, the IRR can be obtained.

The terms 'Risk Ratio', 'RR', *RR(t)* and *RR(S)* refer in the usual and customary sense to the likelihood of developing breast cancer. A RR can compare one group, with a risk factor like high breast density or a genetic mutation, to another group without the risk factor. The RR is the instantaneous prevalence of the disease or malady in the neighborhood of score *S* relative to a cohort average. The RR is given by Equation 6.

In an embodiment of the invention, the RR for cancer refers to the cancer prevalence in the neighborhood of score *S* relative to the cohort average for the type of cancer. In an embodiment of the invention, the RR for breast cancer refers to the breast cancer prevalence in the neighborhood of score *S* relative to the cohort average for breast cancer. In an embodiment of the invention, the RR for a heart arrythmia refers to the arrythmia prevalence in the neighborhood of score *S* relative to the cohort average for the arrythmia. In an embodiment of the invention, the RR for a brain aneurysm refers to the brain aneurysm prevalence in the neighborhood of score *S* relative to the cohort average for the aneurysm. In an embodiment of the invention, the RR for peripheral neuropathy refers to the peripheral neuropathy prevalence in the neighborhood of score *S* relative to the cohort average for the peripheral neuropathy. In an embodiment of the invention, the RR for Crohn's disease refers to the Crohn's disease prevalence in the neighborhood of score *S* relative to the cohort average for Crohn's disease. In an embodiment of the invention, the RR for Celiac disease refers to the Celiac disease prevalence in the neighborhood of score *S* relative to the cohort average for CD.

The terms 'historical data', 'historical data set', refer in the usual and customary sense to data that has been previously measured. Historical data is existing X-ray, CT or MR images (or other data) and does not comprise the step of creating new images of a patient. In an embodiment of the invention, the historical data set is a plurality of entries of 3D image data. In an embodiment of the invention, the historical data set is a plurality of entries of 3D image data in which a Score has been determined such that *N(t)* is available. In an embodiment of the invention, the historical data set is a plurality of entries of 3D image data in which a Score and a CBP have been determined.

The word 'model' refers in the usual and customary sense to a system used to follow or imitate disease or malady outcomes. The model uses a function that mimics data in order to interpret a percentile value and thereby obtain *RR* values. The function can be generated by an AI classifier. In an embodiment of the present invention, the data sets can be historical.

The word 'user' refers in the usual and customary sense to a patient, a medical doctor or other medical professional, a technologist involved in acquiring any one or more of DBT, CT, CTE, CTA, MRI, MRE, or MRA, and any supervisor thereof.

The term 'Cumulative Distribution Function', 'CDF' refer in the usual and customary sense to the probability that a random variable, *I*, is less than or equal to a specific value, x, see Equation 3.

The term 'Empirical cumulative incidence curve' refers in the usual and customary sense to a function that is an estimate of the probability of experiencing a disease or malady *N(t),* see **Section 1.3** and Equation 4B.

The term 'differentiable cumulative distribution function', '*n(t)*'*,* refer in the usual and customary sense to a differentiable function. In an embodiment of the invention, differentiable cumulative distribution function is a mixture of two or more CDFs that approximate *N(t)* and form a continuous function, that is therefore differentiable, see **Section 1.4** and Equation 5.

The term 'Analytic derivative', ' *n'(t)* ', refer in the usual and customary sense to a measure of the rate at which the cumulative distribution function changes with *t*, see **Section 1.5** and Equation 6. In an embodiment of the present invention, the Analytic derivative is a fit of a differentiable cumulative distribution function to the smooth cumulative incidence curve.

The term 'continuous confidence score', '*S(t)*' refer in the usual and customary sense to a level of reliability of predicting a disease or malady outcome, see Equation 11. The continuous confidence score is generated by the model using an AI classifier.

The term 'binning' refers in the usual and customary sense to a data processing technique used to convert continuous numerical data into discrete categories or bins.

The term 'scaling factor' refers to the number of score bins. The IRR can be calculated as a product of *n'(t)* and the scaling factor.

The term 'conversion normalization scoring' refers in the usual and customary sense to the process of standardizing scores from different versions of an AI classifier.

The term 'non-parametric smoothing spline' refers in the usual and customary sense to a type of spline interpolation that aims to find a smooth curve that passes through a set of data points while also balancing the fit to the data with the smoothness of the curve.

The term 'kernel estimator' refers in the usual and customary sense to the process of kernel smoothing for probability density estimation.

The term 'smooth cumulative incidence curve' refers in the usual and customary sense to the probability of a specific event occurring.

The terms 'population', 'cohort' and 'dataset' refer in the usual and customary sense to the number of individuals that are used to train and evaluate the model. The population size is the number of individuals studied in a cohort.

The term 'performance of the classifier' refers in the usual and customary sense to how well the model correctly categorizes data points into different classes.

The term 'characteristics of the classifier' refers in the usual and customary sense to e.g., appearance, reproducibility, transferability, other mobility, and functionality.

The term 'percentile value' refers in the usual and customary sense to the percentage of data points in a set that fall below a specific value. In an embodiment of the present invention, the score can be transformed to a percentile value. In an embodiment of the present invention, transforming the score can to a percentile value enables different AICs to be compared using the same data set.

The terms 'Volume Rendering', 'VR', 'Rendered Volume' and 'RV' refer in the usual and customary sense to computer graphics visualization techniques that create e.g., a 3D image from 3D image data sets, where a typical 3D image data set is a large number of 2D (two Dimensional) slice images. The 2D image slices can be acquired using DBT, CT, CTE, CTA, MRI, MRE, or MRA and can be stored in a data structure. RV techniques include shaded volume, maximum intensity projection (MIP), oblique slicing or multi-planar reformats (MPR), axial/sagittal and coronal slice display, and thick slices (also called slabs).

The terms 'Volume Image', 'Volumetric Image', 'VI', refer in the usual and customary sense to a 3D image generated from a plurality of images of an organ or body part that encompass a three-Dimensional view of the organ or body part, e.g., a 3D image data set.

The terms 'three-Dimensional Computer Tomography Image', '3DCTI', data sets refer in the usual and customary sense to a CT 3D image data set.

The terms 'Volumetric Digital Breast Tomosynthesis Image', 'VDBTI', refer in the usual and customary sense to an FDA-approved imaging technique that creates a 3D reconstruction of the breast from multiple low-dose X-ray images taken at different angles. The VDBTI is a 3D image.

Some or all of the images in a Study can form one or more RVs. For 3D CT modalities, often each individual image in the series corresponds to a RV, but that is not a requirement. For example, a cardiac CT can contain multiple 3D RVs covering the heart, each corresponding to a different point in the cardiac cycle, and all of the images belonging to all of these volumes being grouped into the same series.

The terms 'Digital Breast Tomosynthesis', '3D mammography', and 'DBT' refer in the usual and customary sense to a technique which uses x-rays to create a series of images of the breast. A sufficiently thin-section DBT can allow viewing of breast tissue layer by layer to detect breast cancer more effectively. DBT is especially valuable in women with dense breasts, and can also help differentiate between normal and abnormal tissue, reducing false positives.

The terms 'Computer Tomography scan', 'CT scan', 'Computer Assisted Tomography scan', and 'CAT scan' refer in the usual and customary sense to a diagnostic medical imaging procedure that uses X-rays and computers to create images of organs in the body. It provides cross-sectional views, and can be used to diagnose plan treatments, or monitor disease or malady states. The CT can be used for (i) assessing cardiac chambers, especially determining their size, shape, and thickness; (ii) diagnosing heart enlargement, identifying increased cardiac muscle wall thickness or thinning; (iii) evaluating myocardial diseases, e.g., characterizing conditions affecting the Left Ventricular (LV) myocardium based on its anatomy, function, and enhancement patterns; and (iv) measuring LV wall thickness, e.g., knowledge of normal LV wall thickness is important to diagnose diseases like LV hypertrophy (defined as interventricular septal and/or posterior wall thickness > 11 mm in end-diastole).

The terms 'CT Enterography', or 'CTE' refer in the usual and customary sense to a technique which combines a CT scan with an oral contrast agent that is ingested by the patient to create detailed images of the patient's intestines, digestive tract, bowel wall and surrounding tissues to evaluate inflammation, tumors, obstructions, abscesses, and fistulas. CT and / or CTE can aid in diagnosing e.g., appendicitis, IBD or Crohn's disease by visualizing the small and large intestines. These techniques are able to identify inflammation, and assess the severity of the condition.

The terms 'CT Angiogram', or 'CTA' refer in the usual and customary sense to a technique which combines a CT scan with a contrast dye injected into the patient's bloodstream to create detailed images of the patient's blood vessels. CT and / or CTA can show a pulmonary embolism, a ruptured aneurysm, detect blood leakage into the brain, visualize the size, shape, and location of the pulmonary embolism, and can visualize the size, shape, and location of ruptured and unruptured aneurysms.

The terms 'Magnetic Resonance Imaging', or 'MRI' refer in the usual and customary sense to a non-invasive medical imaging technique that uses MRI with a contrast dye injected into the patient's bloodstream to visualize blood vessels. MRI can be used to detect and assess conditions like pulmonary embolism (e.g., when CTA is contraindicated), aneurysms, stenosis, peripheral neuropathy, blockages in arteries / veins and LV hypertrophy. MRI can provide detailed images of nerves and surrounding tissues, aiding in exact location of nerve damage, differentiate between causes of neuropathy and rule out other conditions, and thereby assist (nerve conduction studies and electromyography) in diagnosis of peripheral neuropathy. DWI, DCE, T1, T2, HBP imaging. When diagnosing Celiac disease, the most characteristic finding is a change in the normal fold pattern of the small intestine. Chronic inflammation in Celiac disease can lead to villous atrophy, causing a decrease in the number and appearance of the folds on MRI.

The terms 'Magnetic Resonance Imaging Enterography', 'MRI Enterography', and 'MRE' refer in the usual and customary sense to a non-invasive medical imaging technique that uses MRI with a liquid contrast agent ingested by the patient and an intravenous contrast agent (gadolinium) to enhance the visualization of blood vessels and tissues in the patient's intestines. The procedure involves drinking a contrast-filled liquid to distend the small bowel, along with an intravenous contrast agent to enhance imaging of blood vessels.

The terms 'Magnetic Resonance Angiography', or 'MRA' refer in the usual and customary sense to a non-invasive medical imaging technique that uses MRI with a contrast dye injected into the patient's bloodstream to visualize blood vessels. MRA can be used to detect and assess in the cerebral cortex, conditions like aneurysms, stenosis, or blockages in arteries and veins.

The terms 'Cardiac Magnetic Resonance Imaging', or 'CMRI' refer in the usual and customary sense to a tool for diagnosing arrhythmias by providing detailed images of the heart's structure and function. CMRI can identify structural abnormalities, assess tissue characteristics, and evaluate heart motion. CMRI can be used for diagnosing the thickness of soft tissues and detecting inflammation in e.g., the cardiac wall.

The terms 'Hepatobiliary phase' imaging or 'HBP' imaging refer in the usual and customary sense to a tool used in a MRI of the liver that specifically assesses the uptake and excretion of contrast agents by hepatocytes. HBP imaging can be used to assist in diagnosing various liver conditions by visualizing how well the liver is functioning and how effectively it is processing the contrast agents.

The terms 'Diffusion-Weighted Imaging' or 'DWI' refer in the usual and customary sense to a tool used with MRI to measure the diffusion of water molecules within tissues. DWI is particularly useful for visualizing and characterizing strokes, tumors, and other brain maladies. The term 'Dynamic Contrast-Enhanced' imaging or 'DCE' imaging, refers in the usual and customary sense to a tool used with MRI that involves acquiring a series of images in rapid succession after administering a contrast agent intravenously. IDCE imaging is used to assess tissue perfusion and vascular permeability. The term 'T1' imaging and 'T2' imaging refers in the usual and customary sense to a tool used with an MRI scan that emphasizes differences in tissue T1 and T2 relaxation times, which is how long it takes for tissues to recover their longitudinal or transverse magnetization after being excited by a radiofrequency pulse. T1 and T2 imaging can be used to visualize normal anatomy in the brain and musculoskeletal system. T1 and T2 imaging can also be used to detect certain maladies such as cancer, myocardial edemas, and multiple sclerosis.

The terms 'Electrocardiogram', 'ECG', and 'Cardiac Sonograph' refer in the usual and customary sense to a recording of the heart's electrical activity through a cardiac cycle. ECG is a graph of electrical activity (in volts) versus time. The ECG is measured using electrodes placed on the skin in and around the chest area. Generally, a thickening of the cardiac wall will result in lower frequency resonances dominating over higher frequency resonances. In this manner, an ECG can be used to diagnose the thickness of soft tissues and detecting inflammation in e.g., the cardiac wall.

The term 'anatomical characteristic' includes the group consisting of one or more of abdomen, bowel, breast, brain, LV, adrenals, retina, pituitary, prostate, spine, chest, shoulder, trapezius, arm, elbow, wrist, finger, pelvis, hip, fibula, knee, tibula, ankle, foot, neck, head, temporomandibular junction, face, dentition, and sinus. The anatomical characteristic can include the Body Part Examined. An anatomical characteristic can be either natural or pathologic. A natural anatomical characteristic of a patient can be the presence of a cyst. A pathologic anatomical characteristic of a patient can be the presence of e.g., a malignant tumor, an arrhythmia, a bowel disorder, or an aneurysm.

The term 'anatomical feature' refers in the usual and customary sense to a medical condition, e.g., whether a lump in the breast, an aneurysm, abdominal pain, an arrhythmia, a fracture in a given image or volume. An anatomical feature is pathologic and can be growth of malignant tissue, a brain aneurysm, inflammatory bowel disease, an arrhythmia, a LV hypertrophy, a fractured fibula, a herniated disc, and urethral bleeding.

The terms 'disease', and 'malady' refer in the usual and customary sense to a medical condition in a human in which a structure or a function is altered such that normal structure or function is deleteriously affected.

The term 'disease-based characteristic' refers in the usual and customary sense to a distinctive feature, or symptom associated with the disease or malady. A disease-based characteristic can be associated with the type of disorder or disease or malady being evaluated, e.g., a diagnosis of breast cancer, a diagnosis of osteoporosis, a diagnosis of a brain aneurysm, a diagnosis of IBD, a diagnosis of Crohn's disease, a diagnosis of Celiac disease, a diagnosis of an arrhythmia, a diagnosis of LV hypertrophy, a diagnosis of prostate cancer, diagnosis of a malignant tumor of glial tissue. That is, the disease-based characteristic can include the Body Part Examined, e.g. breast, prostate, brain, heart, spinal cord, sensory nerves, motor nerves, autonomic nerves, or glial tissue. A disease-based characteristic can be something caused by or relating to disease. It can refer to a process, condition, or tissue that is abnormal due to disease. That is, the disease-based characteristic can be pathologic.

The phrases 'Neural Networks', 'NN' or 'NN protocol' refer in the usual and customary sense to a protocol to generate a classifier and thereby an Analytic derivative of a data set. In an embodiment of the present invention, the data sets can be historical.

The term 'anatomical region' refers in the usual and customary sense to an area of a body, e.g., the breast, the prostate, the brain, the heart or parts thereof, e.g., the LV.

The left ventricle (LV) is the most muscular and powerful of the heart's four chamber, and receives oxygenated blood from the left atrium and then pumps the oxygenated blood out to the rest of the body under high pressure. Thickening of the LV can lead to an inability of the heart to pump blood effectively. LV hypertrophy is often an adaptation to increased workload on the heart, due to high blood pressure or valve problems. LV hypertrophy can go undetected in patients until failure occurs at a point where the window for treatment is severely circumscribed.

When a heart valve is severely damaged or diseased, and the damage is causing noticeable symptoms or putting significant strain on the heart, a heart valve can be replaced by an artificial heart valve (e.g., a transcatheter aortic valve replacement). Diagnosis of a damaged heart valve often involves listening for murmurs with a stethoscope, ECG, stress tests, CT scans, and MRI to assess the severity of the damage to the heart valve and its impact on the heart's function. Diseases or maladies of the heart include aortic stenosis (a narrowing of the aortic valve which restricts blood flow from the heart to the aorta), aortic regurgitation (leakage of blood back into the heart from the aorta), mitral stenosis (a narrowing of the mitral valve which restricts blood flow from the lungs to the heart), mitral regurgitation (leakage of blood back into the left atrium from the left ventricle), and LV hypertrophy (where the muscle wall of the heart's left ventricle is abnormally thick).

The phrase 'Peripheral Neuropathy' refers in the usual and customary sense to a malady that affects the nervous system disrupting normal nerve signals from the brain via the spinal cord to the rest of the body. Peripheral neuropathy, results in a range of symptoms, most commonly in the hands and feet. Peripheral neuropathy can affect sensory nerves which transmit information about touch, temperature, pain, and position to the brain. Damage to sensory nerves can cause numbness, tingling, pain, and difficulty sensing temperature changes or pain. Peripheral neuropathy can affect motor nerves which transmit information and control muscle movement. Damage to motor nerves can lead to muscle weakness, cramps, and difficulty with coordination. Peripheral neuropathy can also affect autonomic nerves which regulate involuntary functions like heart rate, digestion, and bladder control. Damage to autonomic nerves can cause problems with the heart, digestion, and bladder. Peripheral neuropathy can result from genetic factors, nutritional deficiencies, diabetes, injuries, infections, autoimmune diseases, toxins, and some medications. For example, nerves can be damaged due to high blood sugar levels, trauma, pressure, viral or bacterial infections, autoimmune diseases, such as rheumatoid arthritis or lupus, toxins, including heavy metals, medications including chemotherapy drugs and nutritional deficiencies, such as low levels of some B vitamins.

The phrases 'brain aneurysm' or an 'aneurysm' refer in the usual and customary sense to a bulge or ballooning in a blood vessel in the brain. An aneurysm can be caused by a weakening of a blood vessel wall in the brain. This can be a saccular aneurysm, a fusiform aneurysm, or a mycotic aneurysm. A brain aneurysm is often asymptomatic until it ruptures. Once ruptured, causing bleeding in the brain, it results in pressure on the brain, severe headache, nausea, vomiting, stiff neck, vision changes, seizures, loss of consciousness, stroke or death. An unruptured aneurysm can be asymptomatic or cause headaches, or vision changes. Depending on the location, the aneurysm can cause pain above or behind the eye, for example if it presses on nerves or on neural tissue. MRI, Magnetic Resonance Angiography, and CT can be used to identify a patient who without administration of an ameliorating drug will incur an aneurysm. A bulge in a blood vessel, can cause the blood vessel wall to expand or distend outwards. In an embodiment of the observation of a blood vessel that distends outwards by greater than approximately 10% compared to the blood vessel's normal size, can be symptomatic of an aneurysm.

The phrases 'heat arrhythmia', 'dysrhythmia', or an 'arrhythmia' refer in the usual and customary sense to an abnormal heart rhythm. An arrythmia is any heart rhythm that deviates from the normal, healthy pattern. This can involve the heart beating too fast, too slow, or irregularly. This can involve tachycardia, when a patient has a rapid heartbeat, often above 100 beats per minute; bradycardia, when a patient has a slow heartbeat, often below 60 beats per minute; or an irregular rhythm, when a patient's heart beats with an uneven or unpredictable pattern. Arrhythmias occur when the electrical signals that control the heartbeat don't function properly. In a normal heartbeat, electrical impulses travel through the heart in a specific sequence, ensuring the heart chambers contract and pump blood effectively. Arrhythmias arise when these electrical signals become disrupted, either due to issues with the cells that generate the signals, or problems with the pathways through which the signals travel. An arrhythmia can be asymptomatic. Symptoms can vary widely and can include palpitations, shortness of breath, dizziness, lightheadedness, fainting, fatigue, and chest pain. An arrhythmia can be detected through various tests, including an ECG, Holter monitors, event monitors, stress tests, MRI, MRA, and CT.

A 'cell proliferative disorder of the breast' is a cell proliferative disorder involving cells of the breast. Methods of the present application can be used to diagnose breast cancer or cell proliferative disorders of the breast and/or interpret previously measured MRI scans of patient's breasts. Cell proliferative disorders of the breast can include breast cancer, a precancer or precancerous condition of the breast, and malignant growths or lesions of the breast.

The phrase 'reliable ground truth' means in the usual and customary sense verified, factual, and objective data used as the definitive correct answer to at least train, test, and/or validate AI models and systems.

The phrase 'Artificial Intelligence' or 'AI' means in the usual and customary sense a program that performs tasks to be trained and generate output consistent with reliable ground truth by applying machine learning techniques to large collections of data sets. An AI classifier is a model that categorizes data. An AI inference engine is software that runs trained machine learning models to make predictions from data. Unlike the training phase, an AI inference engine is optimized for low-latency, high-throughput, and efficient, real-time performance.

### DETAILED DESCRIPTION OF THE INVENTION

Modem AI-based computer-aided image classification systems, e.g. for disease / malady (e.g., cancer) detection (e.g., mammography), typically output a continuous confidence score (*S*), given by Equation 11 indicating the likelihood of a patient being diagnosed with a disease / malady (e.g., cancer). In an embodiment of the present invention, *Sₘₐₓ* can be equal to 100. In various embodiments of the present invention, *Sₘₐₓ* can be equal to another value (e.g., *Sₘₐₓ* = 1).$S ∈ \left[0, {S}_{max}\right]$

While *S* correlates with likelihood of the disease / malady (e.g., cancer), it is not an absolute likelihood. The actual likelihood of the disease / malady (e.g., cancer) for a given *S* depends on the prevalence and the performance and characteristics of the classifier. Scores can easily be calibrated such that they correspond to percentiles in a representative patient population, so that e.g. 10% of all patients have a score between e.g. 0 and 10% and 10% have a score between 10% and 20%, and so on. But even then, the score does not directly translate to a risk.

### Perfect Classifier Example

In an embodiment of the present invention, a Perfect Classifier can be used to generate a confidence score for a 3DCTI, where the Cohort Baseline Prevalence (e.g., AUC =1) of one per cent (1%) can be chosen. In such a situation, all disease / malady (e.g., cancer) cases receive a score S in the top 1 % of the scale, e.g., between 99 - 100, while every non- disease / malady (e.g., cancer) case receives a score S < 99. Consequently, the absolute disease / malady (e.g., cancer) probability for a S ≥ 99 is 100 %, and the absolute disease / malady (e.g., cancer) probability S < 99 is 0 %.

### Real-World Classifier Example

In an embodiment of the present invention, a Real-World Classifier can be used to generate a confidence score for a 3DCTI, where the separation is imperfect (e.g., AUC ≈ 0.9). In such a situation, the likelihood of finding a disease / malady (e.g., cancer) in a patient with score 95 (or even a score of 50) is greater than zero and the likelihood of finding a disease / malady (e.g., cancer) in a patient with score 99.9 is less than 100%, but the likelihood of finding a disease / malady (e.g., cancer) in a patient is not immediately obvious. Furthermore, if a new, improved version of the model is closer to the perfect classifier, i.e. has a higher AUC, then the likelihood of finding disease / malady (e.g., cancer) in a patient is still not immediately obvious. The Real-World Classifier example underscores that, while the score ***S*** orders examinations by suspicion, additional processing is required before a clinician is required to interpret a specific numeric score value.

In an embodiment of the present invention, in routine practice two (2) population-level curves are commonly inspected to interpret a specific numeric score value, a Complementary Cumulative Distribution Function (CCDF) and a Receiver-Operating-Characteristic curve (ROC curve).

### CCDF

In an embodiment of the present invention, the CCDF survival or exceedance curve given by Equation 12, can be inspected to answer the question 'what fraction of patients would be recalled if we set the cut-off at x'?$\overline{F} \left(x\right) = P \left(S>x\right) ,$

### ROC (Receiver-Operating-Characteristic)

In an embodiment of the present invention, the ROC curve generated by plotting the CCDF of a disease / malady (e.g., cancer) cases (true-positive rate) against the CCDF of non-disease / non-malady (e.g., non-cancer) cases (false-positive rate) can be used with threshold sweeps from high to low to interpret a specific numeric score value.

In various embodiments of the present invention, the CCDF and ROC curves can be used for selecting operating thresholds and benchmarking model discrimination. However, the CCDF and ROC curves integrate over score intervals and thus cannot reveal the individual RR or AR likelihood at a specific score such as for example at 87 or 95.

Further, in decision-support contexts, where the algorithmic output is intended to inform the medical practitioner and /or the radiologist's judgement, interpretability of the meaning of the continuous score is required. Further, reducing a continuous score to a single binary cut-off is sub-optimal, because it suppresses nuanced gradations of risk that can guide follow-up imaging or biopsy recommendations.

In addition, when the CNN is retrained or updated, its score distribution changes; a score of 90 in version 1 can correspond to a markedly different disease / malady (e.g., cancer) risk than a score of 90 in version 2, complicating interpretation of the score.

The existing calibration techniques (e.g. Platt scaling, or isotonic regression) can be used to transform S into an average probability over a neighborhood of scores. However, existing calibration techniques do not provide the IRR. That is, the local slope of the cumulative incidence curve of existing calibration techniques is highly sensitive to arbitrary binning choices. In addition, the outputs of the existing calibration techniques are highly sensitive to arbitrary binning choices. Simple histogram look-ups are piece-wise constant and therefore non-differentiable. The absence of use of a continuous function precludes generating derivative-based risk ratios. In various embodiments of the present invention, Analytic derivative-based risk ratios can be introduced to remedy these deficiencies as outlined in this invention.

### DETAILED DESCRIPTION OF THE INVENTION

While *S* correlates with the likelihood of a disease or malady, it is not an absolute likelihood. The actual likelihood of the disease or the malady for a given *S* depends on the prevalence and the performance and characteristics of the classifier. Scores can easily be calibrated such that they correspond to percentiles in a representative patient population, so that e.g. 10% of all patients have a score between e.g. 0 and 10% and 10% have a score between 10% and 20%, and so on. But even then, the score does not directly translate to a risk.

The present invention builds upon this prior work, addressing limitations by generating the RR, the AR and the IRR.

### System Overview

In an embodiment of the present invention, the system includes a programmable processor executing code to (i) receive a digital mammogram; (ii) execute a neural network producing raw score s ∈ [0, 1] of the digital mammogram; (iii) transform *S* to a percentile *t=100·F_raw(s)* via an empirical CDF of scores (In an embodiment of the present invention, the Scores can be historical); (iv) compute *RR(t)* and optionally *P(C = 1* | *S=t)* using pre-computed parameters (*w*, *α₁*, *β₁*, *α₂*, and *β₂*), and (v) output a structured report and or graphical gauge.

### Curve-Fitting Procedure

In an embodiment of the present invention, the system includes Curve-Fitting Parameters that can be obtained once per population as follows: (i) assemble a dataset of *N* mammograms including the subsequent cancer outcome for each *N*; (ii) 'bin scores' into percentiles between 0 to 100 and accumulate cancer counts; (iii) minimize a weighted least-squares objective as in **section 1.7;** (iv) store the parameters in persistent memory.

In an embodiment of the present invention, alternative smoothers such as cubic splines or kernel density estimators can replace the two-Beta mixture disclosed in **section 1.5** and **section 1.8** without departing from the invention. An example showing the implementation of the curve fitting procedure in python code is given in **section 1.7,** using percentile-normalized score data of a patient cohort.

### Clinical Interpretability

In an embodiment of the present invention, *RR(S)* has units of 'times baseline'. For example, RR = 8 indicates an eight-fold higher risk than average, independent of the prevalence in the population. Absolute risk *P(C=1* | *S)* aids decision-making and triage (e.g., follow-up imaging for P > 10 %).

### Model Versioning and Stability

In an embodiment of the present invention, because of the mapping to actual probabilities, interpretation of *RR* values is largely invariant to network retraining. This property enables continuous model improvement without re-educating clinicians on new numeric scales.

### Hardware Implementation

In an embodiment of the present invention, the method can be implemented in software, firmware, ASIC, or FPGA. Storage of the calibration table can reside locally or be queried via secure API.

### Example 1

In an embodiment of the present invention, a historical data set of 91,008 breast mammograms were analyzed for which the Cohort Baseline Prevalence (b) was found to be approximately 0.28 % (i.e., 256 cancers were confirmed in the historical data set), where approximately means plus or minus two (2) percent (i.e., plus or minus 0.005 %). As outlined above, two-Beta curves were used to fit to the Score. The fitting produced the following parameters *w* = 0.14; *α₁ =* 1.0; *β₁* = 0.29; *α₂ =* 14.7; *β₂ =* 0.31. Unexpectedly, the two-beta curves closely track the observed true-positives count. An advantageous effect observed was that using the two-beta curves it was possible to closely track to the observed true-positives count. Unexpectedly, at percentile *t* = 99.8 the system outputs a RR *(RR)* of approximately 75, and AR (absolute cancer probability) of approximately 17%, where approximately means plus or minus two (2) percent. An advantageous effect observed was that at percentile *t* = 99.8 the system outputs a RR of approximately 75. Another advantageous effect observed was that at percentile *t* = 99.8 the system outputs an AR of approximately 17%.

### Baseline and Monitoring

Patients who undergo serial CT scans (for example, cancer patients who get periodic CTs) can have their RR, AR and IRR for a disease or malady monitored over time using this invention. Clinicians can observe if a patient's RR, AR or IRR of for example, breast cancer is increasing significantly and take action.

### Population Health and Research

On a larger scale, hospitals or health systems can apply this tool to their archive of CT images to gather epidemiological data on e.g., RR, AR and IRR of a specific disease or malady. Because the method is automated and scalable, it can scan through thousands of prior CTs to establish an Analytic derivative for various populations.

Breast cancer is the most common nonskin malignancy in women. In North America, Europe, and Australia, 6% of women develop invasive breast cancer before the age of 75. Risk factors include increasing age, alcohol consumption, estrogen load, infertility, absence of breastfeeding, early menarche, and late menopause. Further, a number of genetic mutations have been identified including BRCA1, BRCA2, MEN1, Li Fraumeni syndrome, Peutz Jegher syndrome, Cowden syndrome, and ataxia telangiectasia. Most breast cancers are classified as adenocarcinomas. Breast sarcomas, malignant phyllodes tumors, breast lymphomas and fibromatosis are non-epithelial breast neoplasms. Papillary lesions and adenomatous lesions are benign breast neoplasms.

In healthy breast cells the HER2 (human epidermal growth factor receptor 2) is a protein that regulates cell growth, division, and repair. The hormone estrogen binds to the Estrogen Receptor (ER) and form a complex which binds to Estrogen Response Elements, a sequence in the genome. In this manner the complex acts as a transcription factor to regulate gene expression. Accordingly, the estrogen-ER complex plays a critical role in various physiological processes, including those related to the reproductive system, bone health, and cardiovascular function. Progesterone binds to the Progesterone Receptor (PR) and forms a complex which binds to Progesterone Response Elements. The progesterone-PR complex acts as a transcription factor and recruits other proteins to regulate gene expression. This process is crucial for various reproductive functions, including preparing the uterus for pregnancy, maintaining pregnancy, and regulating breast development. Three (3) molecular biomarkers (ER, PR, and HER2) are routinely evaluated in invasive breast cancers.

HER2-positive metastatic breast cancer (indicated herein as HER2⁺) is a type of breast cancer characterized by the presence of excess HER2 protein in the cancerous cells, which promotes the growth and division of the cancerous cells. About 15-20% of breast cancers are HER2⁺. Further, HER2⁺ cancerous cells grow and spread more quickly than other types of breast cancer. ER and PR are located on or in breast cancer cells and act as binding sites for estrogen and progesterone, respectively. ER and PR can promote cancer cell proliferation, particularly through interactions with other signaling pathways. If cancer cells express the ER or PR receptors, they are considered Hormone Receptor-positive (indicated herein as HR⁺). Cancer cells that are HR⁺ are likely to be stimulated to grow by the presence of estrogen and/or progesterone.

A cancerous tumor that is 'HER-2 single' means the tumor is either HER2⁺ or HR^{+,} but not both HER2⁺ and HR⁺. A cancerous tumor that is 'HER-2 double' is characterized by the presence of both hormone receptors (HR⁺) and HER2 overexpression (HER2⁺). Half of all HER2⁺ breast cancers are also HR⁺ (and amount to about 10% of all breast cancers). Cancerous tumors that are HR⁺ and HER2⁻ (i.e., HR⁺ but not HER2⁺) account for around 70% of all breast cancers and are the most common subtype of breast cancer. HR⁺ / HER2⁺ breast cancer is generally more aggressive than HR⁺ / HER2⁻.

Staging of breast tumors is performed according to the TNM system published by the American Joint Committee on Cancer (AJCC) / Union for International Cancer Control (UICC). Mammography, ultrasound MRI and CT can be used to identify breast cancer. A CT scan can for example be used to show diffuse thickening of the skin, asymmetry in the breast density, and axillary lymphadenopathy (swelling or enlargement of lymph nodes, e.g., in the armpit).

IBD is a chronic disease that can affect any part of the digestive tract, from the mouth to the anus causing inflammation of the digestive tract. IBD affects 1% of the population in the U.S. IBD can manifest as Crohn's disease (which can occur anywhere in the digestive tract, but is not limited to the lining) or Ulcerative Colitis (e.g., inflammation of the lining of the large intestine and rectum, causing ulcers). Fewer than 200,000 cases of Crohn's disease are diagnosed in the U.S. every year. IBD is characterized by tissue damage, often causing symptoms like diarrhea, abdominal pain, and weight loss. The precise cause of IBD is unknown and can last for the patient's entire life. Based on epidemiologic studies, a combination of genetic, immune, and environmental factors may play a role. Steroids and immunosuppressants can be used to slow the progression of the disease.

IBD is characterized by inflammation. A CTE scan can localize the site(s) of inflammation. The site of inflammation can be visualized on a CTE scan as bowel wall thickening. A CTE scan can also localize complications of IBD, including fistulas, abscesses, and intestinal blockages.

A brain aneurysm can be detected using a CT / CTA scan. The weakening in the blood vessel can sometimes be visualized as a bulging. A CT / CTA scan can show a ruptured aneurysm, detecting blood leakage into the brain, and can also help visualize the size, shape, and location of ruptured and unruptured aneurysms. A CTA scan, can provide more detailed images of blood vessels, making it useful for identifying and characterizing aneurysms, especially for an aneurysm over approximately 3mm in a male adult, and approximately means plus or minus ten (10) percent.

While MRI may not always show all abnormalities, especially in early stages, it can be valuable in detecting changes in the small bowel, and mesenteric structures. The FDA allows 20 ppm of gluten in a product carrying a gluten-free label. The only effective therapy available for people with Celiac disease is a strict, lifelong adherence to a gluten-free diet. However, maintaining a gluten-free existence is quite difficult and expensive, irrespective of the exposure due to the 20 ppm levels allowed by the FDA. Not only can gluten be ingested, but it can also have deleterious effects through inhalation, although apparently for most people not through skin contact. Further, a gluten-free existence is fraught with accidental exposure to gluten when a mislabeled or unlabeled product is consumed, which can result in incomplete recovery and/or relapse from recovery. Celiac disease is characterized by a change in the normal fold pattern of the small intestine. Either, MRI and/or CT scans can be used to detect a dilated small bowel (where dilated means greater than approximately 10 mm in a male adult, and approximately means plus or minus ten (10) percent) or a thickening of the bowel wall (where thickening means greater than approximately 4 mm in a male adult, and approximately means plus or minus twenty (20) percent). In an embodiment of the invention, if the bowel wall thickness of an adult male is greater than approximately 10 mm, then the patient has a Score for Celiac disease of 1 (where approximately when referring to bowel wall thickness means plus or minus ten (10) percent). In an alternative embodiment of the invention, if the bowel wall thickness of an adult female is greater than approximately 9 mm in end-diastole, then the patient has a Score for Celiac disease of 1.

### ADVANTAGEOUS ASPECTS OF THE INVENTION

The described system has several important advantages over prior techniques:

### Improved Accuracy and Reproducibility

A major advantage is the ability to adjust the Analytic derivative data from different NN protocols to be compared. The conversion normalizes scores from different versions of an AI classifier. It means that as a patient changes from one facility to another, their 3D image data set remains meaningful.

### Integration and Workflow

The invention is designed to integrate with existing medical imaging workflows. Radiologists and clinicians can get additional information (e.g., RR, AR or IRR of a disease or malady) without significant extra effort. The outputs can be presented in familiar formats (e.g., as part of the radiology report or as annotated images). This encourages adoption by clinicians, which is essential for opportunistic screening to actually translate into improved patient care.

Moreover, because it uses existing scans, it adds no risk to the patient (no extra procedure, e.g., radiation or chemotherapy treatment) and no inconvenience, which improves patient acceptance.

### Versatility and Extensibility

In an embodiment of the present invention, a focus is on RR, AR or IRR of an initial incidence of breast cancer, the technique can be used for evaluating for the presence of cancer in other organs. In another embodiment of the present invention, a focus is on RR, AR or IRR of an initial incidence of a brain aneurysm, the technique can be used for evaluating for the presence of a brain aneurysm. In an additional embodiment of the present invention, a focus is on RR, AR or IRR of an initial incidence of osteoporosis, the technique can be used for evaluating for the presence of osteoporosis. Further, other diseases or maladies can be amenable to the general methods presented.

### Crohn's disease

For example, 3D image data sets of the gastrointestinal tract, including the small and large bowel for IBD can be used to identify an RR, AR or IRR of occurrence of IBD (and or monitor) in a patient. In an embodiment of the present invention, the data sets can be historical. In an embodiment of the invention, the scoring is based on the thickening of the small intestine at one or more critical regions. The thickening of the bowel wall can be used to generate a Score. In an alternative embodiment of the present invention, the presence of bowel wall thickening together with fistulas, abscesses, and / or intestinal blockages in CTE scans can be used to generate a Score.

### Aneurysms

The concept of using an Analytic derivative of a continuous function to identify an RR is adaptable to detecting aneurysms. That is, 3D image data sets (CT / MRI) of patient's brains for aneurysms, can be used to identify a RR, AR or IRR for the occurrence of an aneurysm (and or monitor) in a patient. In an embodiment of the invention, the scoring is based on the thickening of the blood vessels at a carotid artery to diagnose asymptomatic carotid artery stenosis. In an alternative embodiment of the invention, the scoring is based on the thickening of the blood vessels at a renal artery. In a further embodiment of the invention, the scoring is based on the thickening of a coronary artery. In another further embodiment of the invention, the scoring is based on the thickening of the blood vessels at a carotid artery, a renal artery, a coronary artery and / or one or more of a severe headache, nausea, vomiting, stiff neck, vision changes, and seizures. In an embodiment of the present invention, the thinning or narrowing of blood vessels in the brain can be used to generate a Score. In an embodiment of the present invention, the thinning or narrowing of blood vessels in the brain together with one or more of episodic bleeding in the brain, severe headache, nausea, vomiting, stiff neck, vision changes, episodic seizures, or an episodic loss of consciousness can be used to generate a Score. In an embodiment of the present invention, the thinning of blood vessel can be used to generate a Score for cerebral small vessel disease. In an alternative embodiment of the present invention, the thinning or narrowing of blood vessels in the brain can be used to generate a Score for intracranial atherosclerosis. In an embodiment of the present invention, a narrowing of approximately 50% in the carotid artery can be used to generate a Score for intracranial atherosclerosis (where approximately means plus or minus ten (10) percent). In an alternative embodiment of the present invention, a narrowing of approximately 30% in the carotid artery can be used to generate a Score for intracranial atherosclerosis (where approximately means plus or minus ten (10) percent).

### Peripheral neuropathy.

The concept of using an Analytic derivative of a continuous function to identify an RR is adaptable to detecting peripheral neuropathy. For example, 3D image data sets of the nerves surrounding tissue can be used to identify a RR, AR or IRR for the occurrence of abnormalities like nerve compression, inflammation, or tumors in a patient. In an embodiment of the present invention, the data sets can be historical. In an embodiment of the invention, the scoring is based on the presence of nerve compression. In an alternative embodiment of the invention, the scoring is based on the presence of inflammation surrounding nerves and or nerve endings. In another embodiment of the invention, the scoring is based on the presence of a tumor associated with nerves or nerve endings. In another alternative embodiment of the invention, the scoring in extracranial peripheral nerve disorder is based on the presence of a tumor associated with nerves or nerve endings. In another alternative embodiment of the invention, the scoring in extracranial peripheral nerve disorder is based on the presence of inflammation of the median nerve, the brachial plexus, the ulnar nerve, the sciatic nerve, the lateral femoral cutaneous nerve, and the common peroneal nerve.

### Arrythmia.

The LV wall is thickest near the base and thins towards the apex. The septum is generally thicker than the lateral wall. Men tend to have thicker LV walls than women. The upper limit of normal is higher for men (approximately 13.6 mm) than women (approximately 11.2 mm). LV wall thickness tends to decrease with increasing age. This trend is more pronounced in women. Average LW wall thicknesses are approximately 7 mm.

The concept of using an Analytic derivative of a continuous function to identify an RR is adaptable to arrythmias. In an embodiment of the invention, the scoring for an arrythmia is based on the thickening of the LV wall. 3D CT image data sets of the heart, including the LV can be used to identify a RR, AR or IRR for the occurrence of a LV hypertrophy (and or to monitor) in a patient. In an embodiment of the present invention, the data sets can be historical. In an embodiment of the invention, if the LV wall thickness of an adult male is greater than approximately 11 mm in end-diastole (i.e., the end of the filling phase of the ventricles in the cardiac cycle, before the contraction of the heart), then the patient has a Score for Arrythmia of 1. In an alternative embodiment of the invention, if the LV wall thickness of an adult female is greater than approximately 9 mm in end-diastole, then the patient has a Score for Arrythmia of 1. Approximately, when referring to LV wall thickness is plus or minus ten (10) percent.

### Breast Cancer

Further, the concept of using an Analytic derivative of a continuous function to identify an RR is adaptable to specific types of breast cancers. For example, recurrence of HER2⁺ can be used to identify a RR, AR or IRR for the recurrence in a patient who has previously been diagnosed with HER2⁺. In an embodiment of the present invention, the data sets can be historical. The data could be further fine-tuned for HER-2 single versus HER-2 double (HER-2+ and HR+) (HR+ = Estrogen Receptor +) based on the patients' original diagnoses. Alternatively, data for recurrence of ER-negative and PR-negative breast cancer can be used to identify a RR, AR or IRR for the recurrence in a patient who has previously been diagnosed with ER-negative and PR-negative breast cancer. In an embodiment of the present invention, the thickening of the breast skin can be used to generate a Score. In an alternative embodiment of the present invention, the asymmetry in the breast density can be used to generate a Score. In a further alternative embodiment of the present invention, the thickening of the breast skin, the asymmetry in the breast density and /or the axillary lymphadenopathy can be used to generate a Score.

In an embodiment of the present invention, the thickening of the LV to approximately 11 mm can be used to generate a Score for LV hypertrophy.

In an embodiment of the present invention, the proposed Analytical derivative approach will work with any method of generating a score from 3D image data sets, where there is sufficient data to generate a suitable fit of a continuous function to the data. In an embodiment of the present invention, the data sets can be historical. In an alternative embodiment of the present invention, the proposed Analytical derivative approach will work with any method of generating a score from a data set, where there is sufficient data to generate a suitable fit of a continuous function to the data.

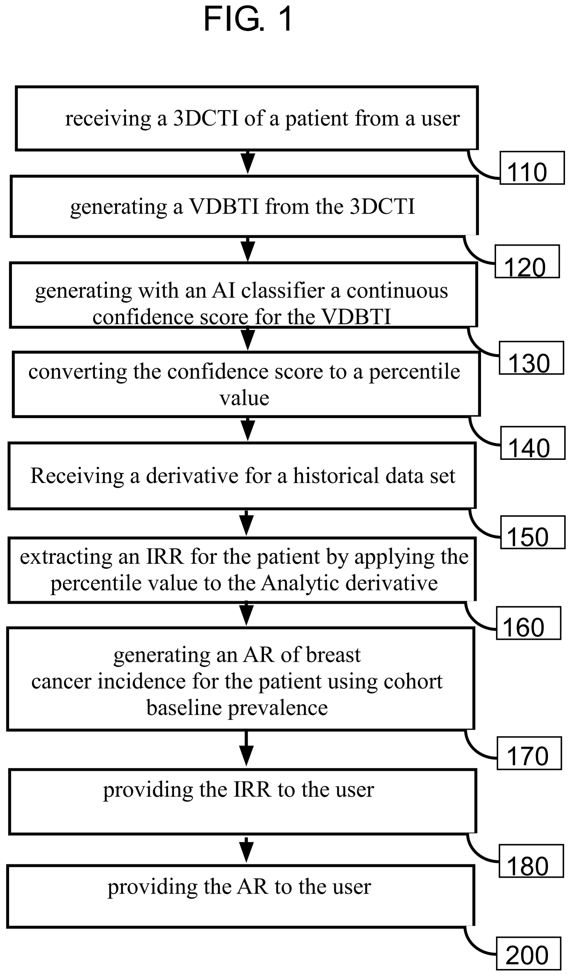

**FIG. 1** depicts a flow chart showing a schematic representation of a method to determine RR and AR for example for breast cancer.

**FIG. 1** depicts a flow chart showing a schematic representation of the method of generating the RR and the AR of breast cancer from a Volumetric Digital Breast Tomosynthesis Image of a patient. In various embodiments of the present invention, at **110,** a user inspects a 3D CTI (Computer Tomography Image) of a patient. The user can generate a VDBTI from the 3DCTI **120.** Based on the VDBTI the user can calculate a confidence score **130.** Further, the confidence score can be converted into a percentile value **140.** The user can receive an Analytic derivative from a data set **150.** In an embodiment of the present invention, the data sets can be historical. The RR for the patient can be extracted by applying the percentile value to the Analytic derivative **160.** The use can also generate an AR of breast cancer incidence for the patient using CBP **170.** The RR and the AR can be provided to the user **180, 200.**

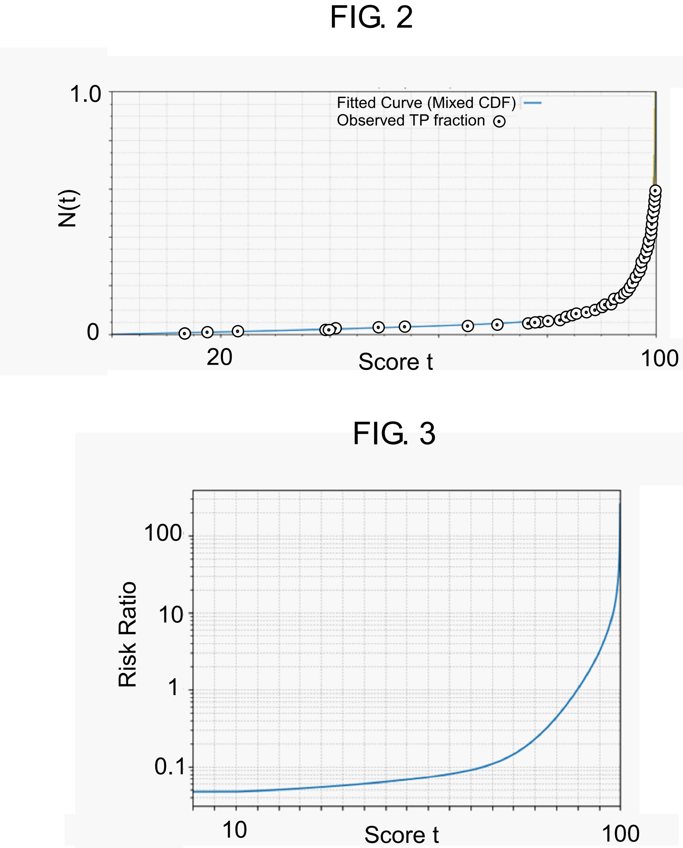

**FIG. 2** shows a plot of (i) the count of true-positive outcome *N(t)* in ground-truth over percentile-calibrated score t (circle) and (ii) a fitted continuous and differentiable mixed-beta function (continuous line) curve. Unexpectedly, the mixed-beta function (continuous line) curve closely tracks the observed true-positives count.

**FIG. 3** depicts the Risk Ratio plotted on a logarithmic axis versus the Score t. Unexpectedly, by employing a mixed-beta differentiable function, the Analytic derivative allows calculation of the risk ratio.

Measurement of BMD, according to steps (A) - (E):

### (A) Automated Segmentation and Labeling

In an embodiment of the present invention, a trained CNN segments the 3D CT scan, identifying and labeling each thoracic and lumbar vertebral body in the scan volume. This segmentation isolates the vertebral trabecular bone for analysis.

### (B) Consistency and Quality Checks

In an embodiment of the present invention, the system performs checks to ensure the segmentation results are anatomically valid. For example: (i) each identified vertebral body forms one contiguous 3D connected component (except in cases where a vertebra is cut off at the scan boundary), and (ii) the vertebrae are in the correct anatomical order. The ordering check may use geometric criteria such as the relative positions, distances, and angles between the centers of mass of consecutive vertebrae to verify that labels increment consistently from cervical/thoracic to lumbar spine. These safeguards detect segmentation errors or mislabeling and allow the system to reject or correct invalid analyses;

### (C) Volumetric Region Of Interest (ROI) Placement for BMD Measurement

In an embodiment of the present invention, for each properly segmented vertebra, the system calculates the vertebra's volume and identifies an internal ROI for measuring trabecular attenuation. The ROI is a three-dimensional spherical volume whose size is a fixed percentage of the vertebra's total volume (for example, 5% of the vertebral body volume). The ROI is automatically placed entirely *inside* the trabecular portion of the vertebra, away from cortical bone boundaries. Critically, the placement algorithm avoids the posterior central region of the vertebral body where the BF and surrounding vascular channels are located (to prevent including the low-density venous plexus or sclerosis around these entry points in the measurement). The system preferentially positions the spherical ROI in an area of relatively low attenuation (lower bone mineral density) within the vertebra's trabecular bone. By using a volumetric ROI (covering many voxels in 3D) instead of a single 2D slice patch, the method reduces the influence of local inhomogeneities and yields a more reliable measure of overall trabecular density;
In an embodiment of the present invention, the ROI's center is chosen such that: (1) the sphere lies completely inside the vertebral body segmentation, (ii) the mean density of bone within the sphere is the lowest obtainable while satisfying (i) (this finds the most osteopenic region of the trabeculae), and (iii) the sphere is located anterior to the BF (thus excluding the posterior vascular channel region). Once placed, the median HU value of all voxels inside this spherical ROI is computed as the representative attenuation for that vertebra. Using the median rather than the mean makes the measurement more robust against outlier voxels (for instance, any small sclerotic spots or artifacts).

### (D) Calibration of Measurements Across Scanner Settings

In an embodiment of the present invention, the system calibrates the extracted HU values to account for differences in scanner models and scanning parameters (in particular, the X-ray tube voltage, or kVp). In CT imaging, the same physical bone can yield different HU values under different tube voltage settings or on different scanner manufacturers/models. In an embodiment of the present invention, the one or more acquisition parameters can be selected from the group consisting of a model of a three-dimensional Computer Tomography scanner used to generate the three-Dimensional Computer Tomography Image and an X-ray tube voltage used to generate the three-Dimensional Computer Tomography Image.

In various embodiments of the present invention, the above variations delineated in (A) - (D) can lead to inconsistency in BMD estimates and potentially misclassification of osteoporosis risk.

In an embodiment of the present invention, to overcome the above variations delineated in (A) - (D), the invention uses a calibration mapping to convert each measured ROI median value to an equivalent value under a reference standard condition (for example, a 120 kVp scan on a reference scanner model). In one embodiment, a linear mapping factor is applied: HUcalibrated *= A* · HUmeasured, where *A* is a calibration coefficient determined for the specific combination of scanner model and kVp. The calibration factors can be derived from a large reference dataset using a phantom-less statistical approach - for instance, by comparing average vertebral attenuation in cohorts scanned at different kVp settings.

In an embodiment of the present invention, by calibrating to a common scale, the system ensures that BMD measurements are comparable across scans taken on different equipment and protocols. This approach obviates the need for inserting physical calibration phantoms in each scan, which is impractical for opportunistic screening.

The system and methods build on known techniques in the field of quantitative CT (QCT) where phantom-based or phantom-less corrections are used to standardize measurements.

### (E) Comparison to Normative Reference Data

In an embodiment of the present invention, the CT scan is of a mammal. In an embodiment of the present invention, the mammal is a human. In an embodiment of the present invention, the human is male. In an embodiment of the present invention, the human is female.

In an embodiment of the present invention, the calibrated trabecular attenuation (BMD surrogate) for each vertebra is then compared against reference curves or databases of normative values stratified by age and sex. Large population studies have established distributions of vertebral trabecular HU values by age group and sex.

For example, normative percentile curves for each vertebral level (e.g., T10, L1, etc.) can be used to determine where a patient's measurement lies in the population distribution. The software determines the patient's percentile (e.g., 20th percentile for age 70 female at L1) or compares against fixed threshold values that correspond to clinical definitions of osteoporosis. In some implementations, the system calculates the patient's values for multiple vertebrae (for instance, all vertebrae fully visible in the scan from T10 through L4). To provide a robust single summary value per patient, the median vertebra's attenuation can be selected - that is, the median of the percentiles across multiple vertebrae is used as the representative BMD measure for the patient. Using the median of multiple vertebral measurements mitigates the impact of any one abnormally high or low vertebra (for example, a fractured or degenerative vertebra).

In an embodiment of the present invention, the chosen value (median of a particular vertebra like L1 or the most median vertebra) is then used to determine the patient's BMD status. The system can output an age/sex percentile ranking and can flag patients below a certain percentile or threshold (for example, below the 12th percentile, which roughly corresponds to osteoporosis prevalence in older adults.

**(F)** In an embodiment of the present invention, the output obtained from steps (A) - (E) for osteoporosis screening based on bone mineral density in the trabecular bone of vertebrae from 3DCT scans can be input to generate a clinically interpretable Instantaneous Risk Ratio. Further when combined with the CBP for osteoporosis an Absolute Risk for osteoporosis can be generated. In an embodiment of the present invention, existing CT scans are analyzed and compared with historical CBP to identify an AR for osteoporosis, and accordingly the method adds no risk to the patient through additional exposure to radiation and no inconvenience, which improves patient acceptance.

Hounsfield Units (HU) and T-scores are distinct non interchangeable measurement systems that relate to bone density. Hounsfield units are physical measures of tissue density i.e., a measure of X-ray attenuation in specific voxels on a CT scan. Hounsfield units are in a standardized, dimensionless scale, where pure water has an HU value of 0, air is defined as - 1000 HU, bone and dense materials, have positive values (e.g., +700 to +3000 HU), while less dense materials like fat have negative values (e.g., -120 to -90 HU). CT artifacts, patient anatomy, and the specific CT scanner settings can influence HU values. In contrast, T-scores are statistical measures from a Dual-energy X-ray Absorptiometry (DXA) scan which can be used to classify bone health relative to a healthy population. T-scores are derived from total BMD measurements of a region in the DXA scan. The T-score represents the number of standard deviations a person's bone mineral density (BMD) is above or below the average BMD of a healthy, young adult of the same sex. DXA scans in patients with degenerative spine disease can result in elevated T-scores. A formula such as T-score = bHU + c is considered to be context-dependent and not typically useful in practice. However, a DXA scan is currently considered the gold standard for diagnosing osteoporosis.

In an embodiment of the present invention, the system and methods can also categorize the patient as having normal BMD, osteopenia, or osteoporosis by comparing to diagnostic cut-offs equivalent to those from DXA based criteria. A T-score from a DXA scan above -1.0 can indicate a patient has normal BMD. A T-score from a DXA scan between -1.0 to -2.5 can indicate mild to moderate bone loss and can result in a diagnosis of osteopenia. A T-score from a DXA scan below -2.5 can indicate low BMD and can result in a diagnosis of osteoporosis.

In summary, the invention combines advanced AI driven image segmentation, intelligent ROI selection, physics-based calibration, and epidemiologically-derived reference comparisons into a cohesive tool for opportunistic osteoporosis screening. It enables an automated, reproducible measurement of vertebral bone mineral density from existing CT scans, allowing identification of patients at risk for osteoporosis without additional dedicated scans. The method is more robust and accurate than prior approaches that used 2D single-slice measurements, because it analyzes a larger volume of bone and accounts for scanner variability and anatomical correctness of vertebral identification and automatically places the ROI. This system can be deployed in various forms, including as a software add-on to hospital PACS systems, a standalone application, or a cloud-based service, as described further below. xxx

Osteoporosis is traditionally diagnosed by measuring BMD via DXA scans, but millions of CT scans taken for other reasons contain bone data that can be used to screen for osteoporosis opportunistically.

Prior research has demonstrated a correlation between CT attenuation of vertebrae and bone mineral density, offering thresholds to identify osteoporosis. For example, routine abdominal CT scans can be used for osteoporosis screening, proposing around 100 HU at L1 as a threshold indicative of osteoporosis risk. Later studies analyzed over 20,000 CT scans and reported normative trabecular attenuation values at L1, suggesting an approximate 90 HU threshold for osteoporosis at that level. In general, patients with low vertebral HU on CT are more likely to have low BMD on DXA.

However, early approaches often relied on manual or semi-automated techniques. The standard practice was to place a single 2D circular or elliptical ROI in the mid-section of one vertebral body (such as L1) on an axial CT slice and read the average HU value. This manual ROI placement is subject to variability. Results can differ depending on the slice level or exact ROI position, because trabecular bone mineral density is not uniform throughout a vertebra.

Small areas of sclerosis can skew the measurement. Indeed, it has been noted that there is significant variation in trabecular attenuation within a single vertebra when measured with a 2D ROI on one slice. Studies using a larger ROI volume can improve reproducibility. Furthermore, differences in CT scanner settings (tube voltage) and models can cause systematic differences in HU measurements of bone.

Previous commentators warned that attenuation in L1 trabecular bone can vary at different tube voltages, cautioning against using unadjusted HU values for osteoporosis screening across scans with dissimilar protocols. In other words, a patient's L1 HU measured at 80 kVp may not have the same attenuation as the same patient's L1 HU at 120 kVp, potentially leading to misclassification of osteoporosis if not corrected. It was further shown that automated opportunistic CT measures can predict future hip fractures.

One known challenge in these automated approaches was the calibration of HU values: early implementations sometimes applied a single conversion factor for all scans, which can introduce error if applied universally across different scanner models.

The present invention builds upon this prior work, addressing resolution of their limitations by introducing more refined calibration and robust ROI selection strategies.

Unexpectedly it was found the integration of advanced computational techniques enhance the accuracy of BMD estimation. It was also unexpectedly found the integration of advanced computational techniques enhance the robustness of BMD estimation. Further, it was unexpectedly found the integration of advanced computational techniques enhances the standardization of BMD estimation. In an embodiment of the present invention, the integration of an advanced computational technique enhances the accuracy of BMD estimation. In an alternative embodiment of the present invention, the integration of an advanced computational technique enhances the robustness of BMD estimation. In another embodiment of the present invention, the integration of an advanced computational technique enhances the standardization of BMD estimation

A deep learning-based CNN segmentation is used to precisely identify vertebral bodies in 3D, followed by anatomical consistency checks to detect and correct labeling errors, ensuring robustness against segmentation inaccuracies present in prior methods.

In an embodiment of the present invention, a 3D spherical ROI, sized as a fixed fraction of the vertebral volume, is strategically positioned using convolution-based optimization to locate the lowest-density region within the trabecular bone while avoiding anatomical artifacts such as the BF. Unlike prior approaches that positioned ROIs at the vertebral center or relied on single-slice measurements, which risk capturing denser cortical bone or structural irregularities, this method ensures a more precise assessment of trabecular attenuation.

In an embodiment of the present invention, the median HU value within the spherical ROI is used instead of the mean, improving measurement robustness by minimizing the influence of outliers such as focal sclerosis, calcifications, or artifacts that can skew the results in conventional averaging-based approaches.

In an embodiment of the present invention, the system selects the most representative vertebra for analysis based on percentile rankings across multiple vertebrae in a given patient. Instead of relying on a single predefined vertebra (e.g., L1) as in prior methods, this approach ensures that the measurement reflects overall trabecular bone health rather than being biased by localized pathology, fractures, or anomalies.

In an embodiment of the present invention, a two-step calibration process adjusts attenuation values for differences in scanner models and X-ray tube voltages (kVp), ensuring consistency across imaging protocols. This phantom-less standardization allows direct comparison of BMD estimates across different scans, eliminating variability introduced by hardware differences and imaging settings.

### System Architecture and Workflow

In an embodiment of the present invention, a software pipeline can process a 3D CT scan (axial image series) and output a BMD report. The pipeline may be integrated into a Picture Archiving and Communication System (PACS) or run on a separate workstation or cloud server. The steps of the method are as follows:

### 1. Input Acquisition

In an embodiment of the present invention, the system receives a CT scan volume that includes portions of the spine. This can be any CT of the chest, abdomen, or spine where at least one vertebral body is fully in view. The DICOM images are loaded into the processing software.

### 2. Vertebrae Segmentation using CNN

In an embodiment of the present invention, the entire 3D volume is passed through a trained convolutional neural network which outputs a segmentation mask identifying voxels that belong to bone (and specifically labels the vertebral bodies). The CNN is trained on a large set of manually labeled CTs to recognize vertebral bodies and differentiate them from other structures. It assigns an identifier to each vertebra (for example T10, T11, ..., L4, etc.) based on learned anatomical patterns (the network can output separate labels for each vertebral level). In some implementations, a two-stage approach may be used: first identify all vertebrae as a class, then apply a labeling scheme to distinguish levels. The result of this step is a set of 3D masks, one for each vertebral body present in the scan, along with an initial label (name/number of the vertebra).

### 3. Segmentation Consistency Checks

In an embodiment of the present invention, the system then evaluates the segmentation for quality:

### 3A. Connectivity Check

In an embodiment of the present invention, for each vertebral mask, the software checks that the mask is one connected 3D region. If a vertebra's mask is fragmented into multiple pieces (which can happen if the CNN mistakenly split one vertebra or if part of the vertebra is missing at scan edges), the system can flag this as an error. In edge cases where a vertebra is at the superior or inferior extreme of the scan range (cut off by the scan boundary), multiple components might appear (e.g., only the top half of a vertebra is present). Those boundary vertebrae can be excluded from analysis, since they are not fully captured. Ensuring single-component segmentation for each vertebra confirms that the CNN correctly identified whole vertebral bodies.

### 3B. Sequential Order and Position Check

In an embodiment of the present invention, the relative positions of the labeled vertebrae are verified to ensure proper ordering (e.g., T12 should be below T11 and above L1, aligned roughly along the spinal axis). The software computes each vertebra's center of mass (or centroid of the mask) in 3D coordinates. Then it checks that as one moves down the spine, the centroids progress consistently without large jumps or reversals. The system may calculate the angle of the line connecting successive centroids and the distance between them to verify a smooth anatomical curve: The angle should be greater than 90 degrees and the distance from one set of vertebrae to the next should not differ by more than a pre-defined factor, e.g. 1.5x.

In an embodiment of the present invention, if two adjacent identified "vertebrae" have centroids that are too far apart or not aligned vertically, it can indicate a missed vertebra or a labeling mistake (e.g., skipping a vertebra number). In an embodiment of the present invention, if the order or count is inconsistent, the software can relabel using expected spacing. In an alternative embodiment of the present invention, if the order or count is inconsistent, the software can mark the case as requiring manual review. This geometric sanity check ensures that each vertebral body is correctly identified by level.

In an embodiment of the present invention, if the segmentation passes these consistency checks, the process continues; otherwise, the system may either correct minor issues (like merge small fragmented components, or adjust a label) or abort processing for that scan and output an error indicating the automated analysis was not confident.

### 4. Vertebral Volume Calculation

In an embodiment of the present invention, for each vertebra that passed validation, the volume (in cubic millimeters or cubic centimeters) of the trabecular region is computed from the segmentation mask. The volume is simply the count of voxels in the mask times the volume per voxel. This volumetric information is used to size the ROI in the next step. In an embodiment of the present invention, the volumetric information can also serve as an indicator (e.g., a very small volume might indicate an incomplete or damaged vertebra).

### 5. Spherical ROI Placement

In an embodiment of the present invention, the system determines a spherical region of interest inside each vertebra for measuring attenuation.

### 5A.

In an embodiment of the present invention, the target ROI volume is set as a percentage of the vertebra's volume (e.g., approximately 5%, where in this range approximately means plus or minus 30%, i.e., 3.5% to 6.5%). In an embodiment of the present invention, the proportion can be adjusted. In an embodiment of the present invention, 5% has been found effective in capturing a representative sample while being small enough to fit entirely within most vertebrae.

### 5B.

In an embodiment of the present invention, an algorithm searches within the vertebral mask to find the optimal location for this sphere. The criteria include:

### 5B(i).

In an embodiment of the present invention, the sphere can fit entirely within the vertebra mask. In an alternative embodiment of the present invention, the sphere fits entirely within the vertebra mask so that none of its voxels fall outside the segmented bone.

### 5B(ii).

In an embodiment of the present invention, the sphere's average density (HU) is as low as possible. In an alternative embodiment of the present invention, an algorithm can scan the interior of the vertebra, evaluating candidate sphere positions to find a location that minimizes mean HU. In another alternative embodiment of the present invention, an algorithm can scan the interior of the vertebra, use an optimization strategy to find a location that minimizes mean HU. In various embodiments of the present invention, this tends to place the ROI in the most least dense (osteopenic) trabecular region of the vertebra.

### 5B(iii).

In an embodiment of the present invention, the sphere can be positioned anteriorly relative to the center of the vertebra. In an embodiment of the present invention, the sphere can be positioned avoiding the posterior third of the vertebral body. In an embodiment of the present invention, the sphere can be positioned where the BF and surrounding venous plexus are located. Positioning the sphere in the above manner is important because the basivertebral venous plexus can appear as a localized low-density or high-density area depending on blood or sclerosis around it, which can otherwise confound the HU measurement.

### 5C.

In an embodiment of the present invention, once the sphere position is chosen, the ROI sphere is defined, and its voxels are recorded.

### 6. Attenuation Measurement.

In an embodiment of the present invention, with the ROI in place, the system extracts all the HU values of voxels inside the spherical ROI. In an embodiment of the present invention, with the ROI in place, the system then calculates the median HU value of these voxels. In an embodiment of the present invention, the median is found by ranking the values and picking the middle one. In an embodiment of the present invention, if there is an even number of voxels, the median is an average of the two central values. The choice of median provides robustness to any outlier values, such as a small patch of sclerosis that might raise the mean but can only occupy a small fraction of the ROI.

In an embodiment of the present invention, the median HU is taken as the representative trabecular attenuation for that vertebra.

### 7. Calibration of HU to BMD Scale.

In an embodiment of the present invention, the raw median HU for each vertebra is then calibrated to a standardized scale. In one embodiment, two calibration factors are applied multiplicatively: one for the scan's kVp, and one for the scanner model. For example, if the scan was done at 100 kVp on Scanner Model X, the system will apply:

### 7A.

In an embodiment of the present invention, the HU_adjusted_for_kVp = HU_median * CkVp, where CkVp=100-120</sub> is a coefficient converting 100 kVp to the 120 kVp reference. In an embodiment of the present invention, if bone appears denser at lower kVp, this coefficient might be <1.0 to scale down, or >1.0 to scale up.

### 7B.

In an embodiment of the present invention, then the HU_adjusted_for_scanner equals HU_adjusted_for_kVp * CModelX , where CModelX is a factor for that specific scanner relative to the reference scanner model. These factors can be determined ahead of time by analyzing large datasets as described earlier. Tables of calibration coefficients for common kVp settings and scanner manufacturers can be stored in the system. The result of this step is a calibrated HU value for each vertebra that represents what the attenuation can be if the scan had been performed under reference conditions. By performing this calibration, the method aligns the measurements with a consistent BMD scale, addressing the known issue that different CT settings yield different attenuation readings.

### 7C.

In an embodiment of the present invention, a more complex function or even a machine learning model can be used for calibration (taking into account one or more of kVp, reconstruction kemel, and patient size).

### 8. Analysis of Multiple Vertebrae (if available):

The system will repeat steps 5-7 (see above and paragraphs [0104] to [0128] of U.S. Provisional Patent Application 63/764,573, filed February 28, 2025) for each vertebral body that is fully within the scan range (e.g., T12, L1, L2, L3, etc., depending on the scan). This yields a set of calibrated attenuation values for the patient's spine. If more than one vertebra is measured, the system can aggregate these to improve reliability. One preferred approach is to identify the most median vertebrae and use it as the patient's representative value.

For example, if T12, L1, and L2 are measured, sort those three values and take the middle value. This approach helps avoid cases where a single abnormally low value (perhaps from a localized bone issue or imaging artifact) can falsely classify the patient as osteoporotic, or a single abnormally high value (perhaps due to degenerative sclerosis in one vertebra) can mask osteoporosis in the others. The system can be configured to use a particular vertebra or a combination. In an example embodiment, the software selects the L1 vertebra if it is present and fully captured; if not, it uses the next available (T12 or L2), or uses an average of the nearest two, etc. The median-of-multiple approach is generally preferred for robustness if multiple levels are available.

### 9. Comparison to Reference Data.

The final calibrated attenuation value (or values for each vertebra) are compared to reference standards. The invention includes a database of normative attenuation values by vertebral level, age, and sex (derived from population studies or clinical data).

For each vertebra measured, the system can determine the patient's percentile. It can also determine an overall percentile or category for the patient. For instance, the system might output:

### 9A.

L1 trabecular attenuation = 55 HU, which is the 8th percentile for a 65-year-old female (significantly below average).

### 9B.

By established criteria, this is consistent with osteoporosis since 58 HU at L1 is below the cut-off for osteoporosis of 68HU as per reference data.

### 10. Output Generation.

The system generates a report or data file with the results. This can include the numerical values (raw and calibrated HU), the reference percentile, and a suggested diagnostic category. It may also include a visual image with the ROI marked on a representative CT slice for review, or a 3D rendering of the spine with color-coding of BMD. In a PACS integration, the results can be sent as secondary capture images or DICOM Structured Reports attached to the patient's study. In a cloud service, the results might be delivered via a web interface or an API to an electronic health record.

Throughout this process, the method is fully automatic. It does not require a technician to place ROIs or calibrate the machine, making it suitable for high-throughput screening of large numbers of CT scans. The results are presented to the radiologist or other physician for confirmation before making a final diagnosis or treatment decision.

### Embodiments.

The invention can be embodied in several forms to fit into clinical workflows:

### Standalone Processing Service

In an embodiment of the present invention, the software operates on a dedicated workstation or server within a radiology department. CT scans from the scanner or PACS are forwarded to this system (for example, via DICOM network transfer). The system processes each scan to compute the BMD metrics and then returns a report. Radiology staff can then review the results and incorporate them into patient evaluations. This standalone system can be a software package installed on existing hardware or sold as an appliance (hardware + software).

### PACS Integration.

In an embodiment of the present invention, the system can be integrated directly into a hospital's PACS or advanced visualization workstation. For instance, when a radiologist opens a spine or body CT in the PACS, they can trigger the "BMD Analysis" which invokes the algorithm on that series. The integration can also be automatic: as soon as a new CT scan is saved to PACS, the system detects if spine vertebrae are present and, if so, performs the analysis in the background. Results can then be attached to the study for the radiologist to see. Integration ensures the tool fits seamlessly into existing image interpretation workflows.

### Cloud-Based Deployment

In an embodiment of the present invention, the analysis is carried out on cloud servers. CT scans (de-identified or via secure VPN) are uploaded to a cloud service that runs the AI algorithm. This is useful for institutions that prefer not to maintain on-premises hardware or for providing the service to many sites. A cloud deployment also facilitates continuous learning: the system can aggregate data from all processed scans to further refine normative databases or improve the CNN through federated learning (if permitted).

Each embodiment leverages the same core algorithm but offers different advantages. The standalone and PACS-integrated versions allow for real-time use within a hospital with minimal data leaving the facility. The cloud version offers scalability and easier updates (since improving the AI model or calibration curves can be done centrally). Importantly, regulatory compliance and data privacy are maintained in each case.

### Examples.

### Opportunistic Osteoporosis Screening

The primary use case is analyzing existing CT scans (taken for any indication, e.g. trauma, pain, cancer staging, etc.) to identify patients with low bone mineral density who might otherwise be unaware of their condition. For example, an older patient gets an abdominal CT for kidney stones; the radiologist runs this tool and discovers the patient's spine shows low BMD, prompting a recommendation for a dedicated bone health evaluation. This opportunistic screening can significantly increase osteoporosis detection rates without additional radiation or scan appointments.

### Fracture Risk Assessment

The system's results can be used alongside clinical risk factors to assess fracture risk. Doctors might use the vertebral attenuation measurement in calculators or scoring systems. A very low HU value in the spine can suggest high fracture risk, leading to interventions (like starting osteoporosis medication or fall-prevention strategies). Research has shown that CT-based bone measures are predictive of future fractures, so this tool can aid in risk stratification.

### Baseline and Monitoring

Patients who undergo serial CT scans (for example, cancer patients who get periodic CTs) can have their bone mineral density monitored over time using this invention. The calibrated values allow comparison across scans even if different machines or protocols were used. This can effectively provide a "CT bone densitometry" track record without dedicated scans. Clinicians can observe if a patient's bone mineral density is dropping significantly and take action.

### Population Health and Research

On a larger scale, hospitals or health systems can apply this tool to their archive of CT images to gather epidemiological data on bone health. Because the method is automated and scalable, it can scan through thousands of prior CTs to establish normative BMD ranges for various populations.

Public health researchers can use such data to study osteoporosis prevalence in different demographic groups or the effect of certain treatments on bone mineral density incidentally.

### ADVANTAGEOUS ASPECTS OF THE INVENTION

The described system has several important advantages over prior techniques:

### Automation and Efficiency

In an embodiment of the present invention, the system eliminates the need for manual ROI placement, reducing labor and inter-observer variability. Once deployed, it can process scans rapidly and consistently, ensuring that no potential osteoporosis case is missed due to human oversight. This is crucial given the large number of CT scans performed; even a modest fraction being analyzed can yield many detections of previously unknown osteoporosis.

### Improved Accuracy and Reproducibility

By using a 3D ROI and median value, the measurement is more reproducible across different slices or different scans of the same patient.

In an embodiment of the present invention, the approach is less sensitive to small local anomalies in the bone. The consistency checks on segmentation also ensure the anatomy is correctly identified, preventing gross errors.

### Sensitivity to Low BMD

In one embodiment of the present invention, the strategy of targeting the lowest-density region in the trabecular bone (while avoiding artifacts) makes the measurement more sensitive to osteoporosis. Traditional single-slice methods might miss such low-density spots or dilute them with higher-density bone, yielding false-negatives. Our method effectively seeks the "weakest point" of the bone's internal structure to gauge overall bone health, which correlates with fracture susceptibility.

### Calibration Across Scanners

A major advantage is the built-in calibration that allows data from any CT scanner or protocol to be interpreted on the same scale. This means the tool can be deployed across different hospitals with varying equipment and still produce comparable results. It also means that as a patient goes from one facility to another, their CT-based BMD measurement remains meaningful. Prior art either ignored these differences or required phantom scans; our approach makes it seamless and based on statistical knowledge gleaned from real-world data.

### Integration and Workflow

The invention is designed to integrate with existing medical imaging workflows. Radiologists and clinicians can get this additional information without significant extra effort. The outputs can be presented in familiar formats (e.g., as part of the radiology report or as annotated images). This encourages adoption by clinicians, which is essential for opportunistic screening to actually translate into improved patient care.

Moreover, because it uses existing scans, it adds no risk to the patient (no extra radiation) and no inconvenience, which improves patient acceptance.

### Versatility and Extensibility

While the current focus is on vertebral bodies for osteoporosis, the same framework can be extended to other bones (e.g., femoral neck on hip CTs) or other clinical metrics (like detecting high density in bone metastases or quality of bone grafts). The modular design (segmentation → ROI → measure → calibrate → compare to reference) can be adapted, demonstrating the flexibility of the approach.

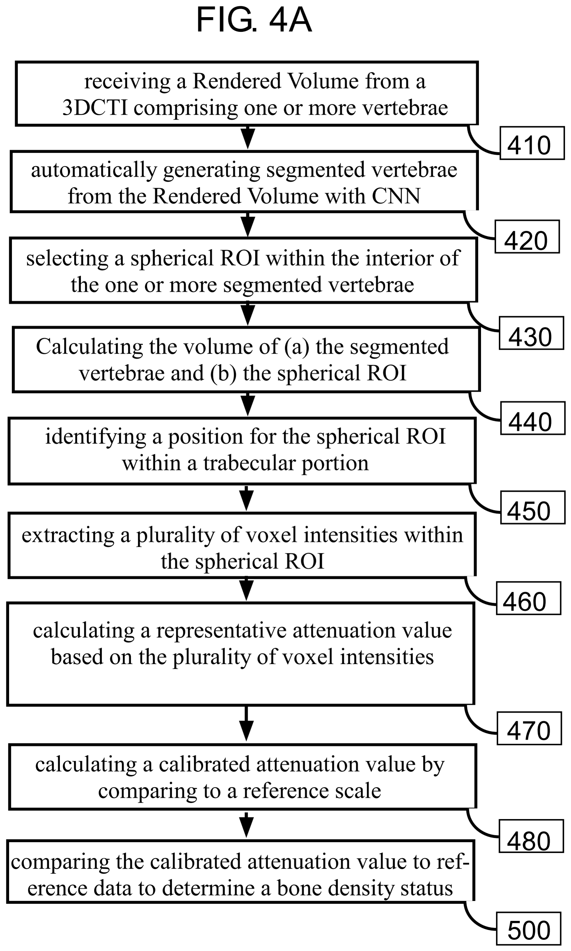

**FIG. 4A** depicts a flow chart showing a schematic representation of a method to determine bone mineral density status of a segmented vertebra. In step **410** a three-Dimensional Computer Tomography Image (3DCTI) comprising one or more vertebrae is received. In step **420** one or more segmented vertebrae are automatically generated from the 3DCTI with a Convolutional Neural Network. In step **430** a three-Dimensional Spherical Region of Interest (3DSROI) is selected where the 3DSROI is within the interior of the one or more segmented vertebrae. In step **440** a volume of the three-dimensional Spherical Region of Interest (3DSROIV) is calculated. In step **450** a position for the 3DSROI is identified, where the 3DSROI lies entirely within a trabecular portion of the one or more segmented vertebrae. In step **460** a plurality of voxel intensities within the 3DSROI are extracted. In step **470** a representative attenuation value based on the plurality of voxel intensities is calculated. In step **480** a calibrated attenuation value is calculated based on comparing the representative attenuation value to a reference scale to compensate for differences in one or more acquisition parameters of the 3DCTI. In step **500** the calibrated attenuation value is compared to a population-based reference data to determine a bone mineral density status of the one or more segmented vertebrae.

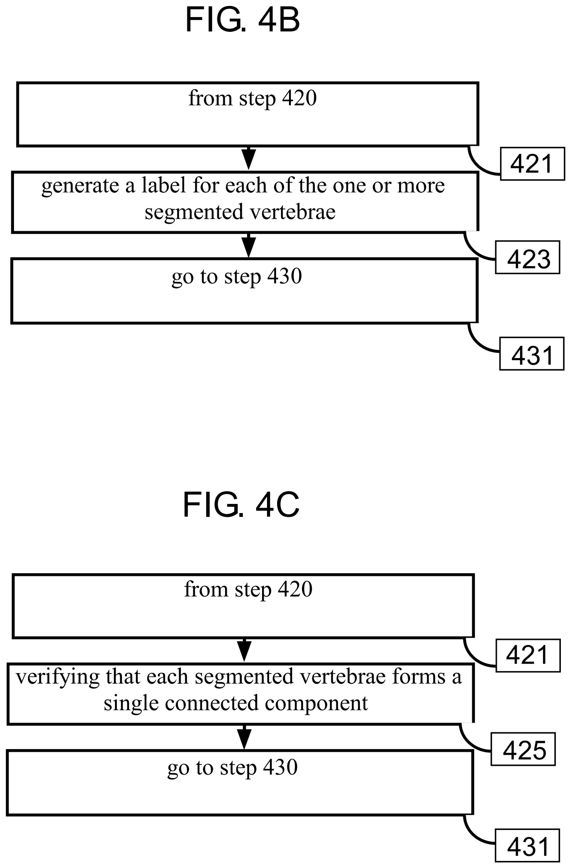

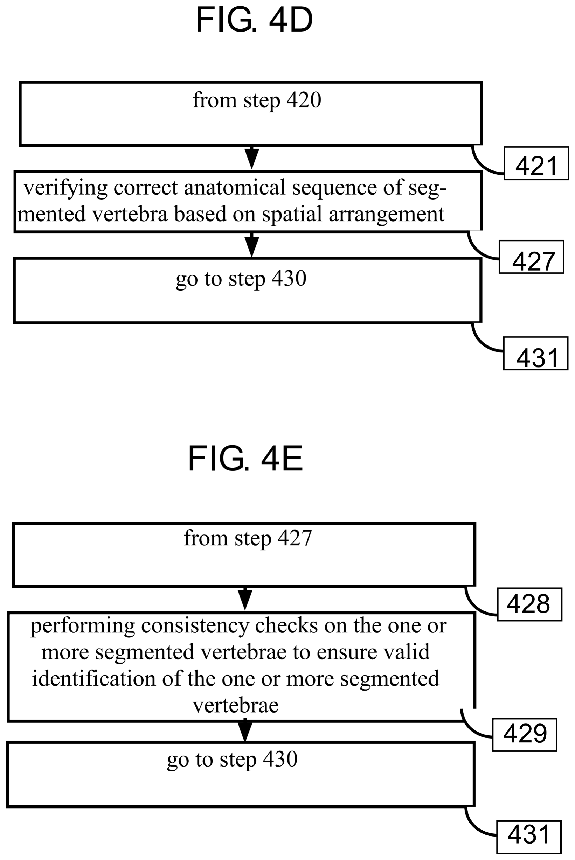

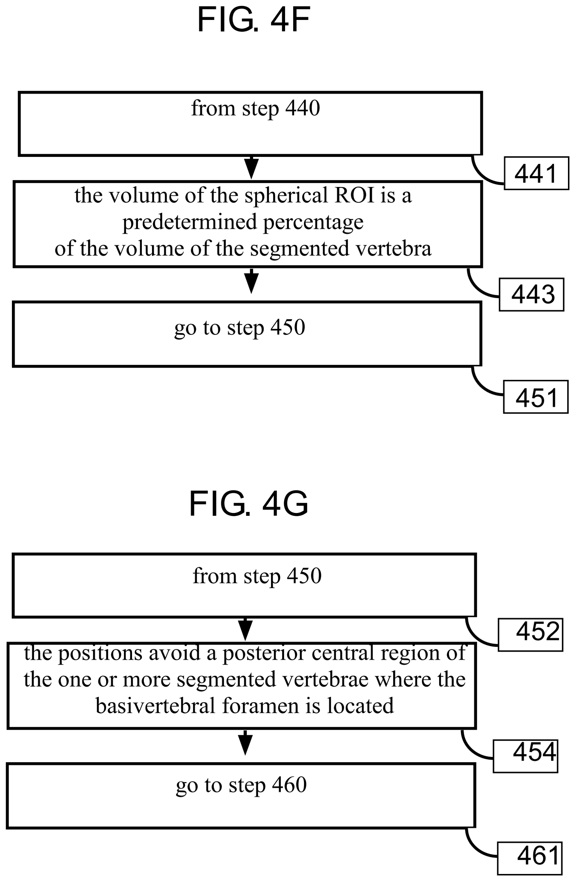

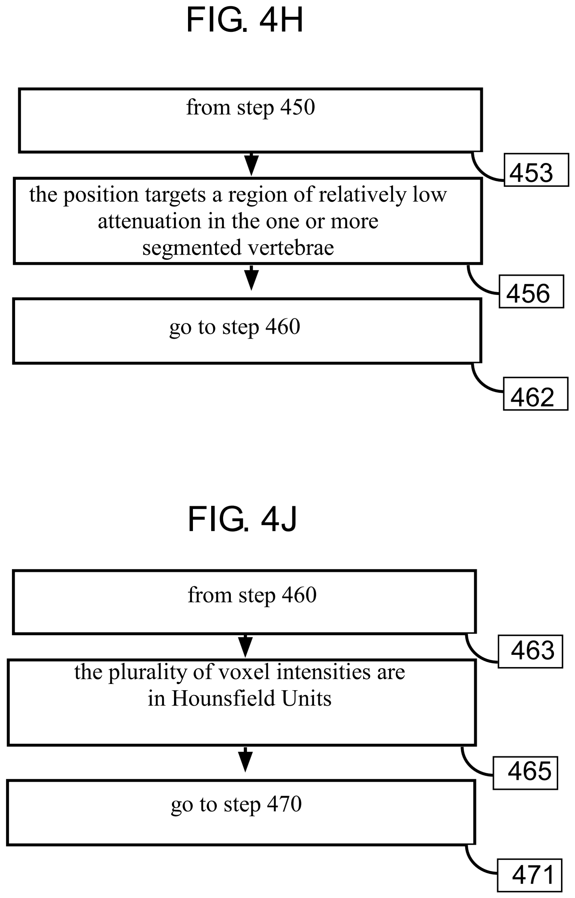

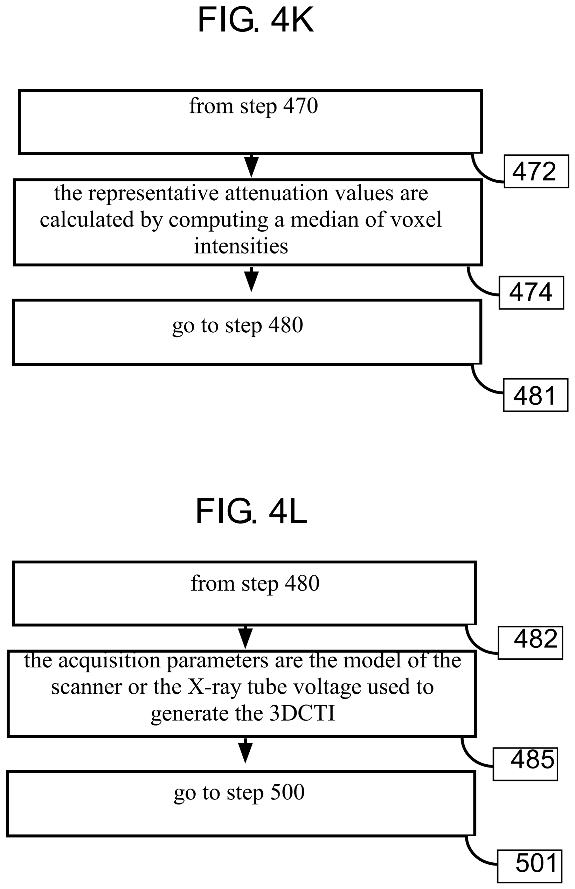

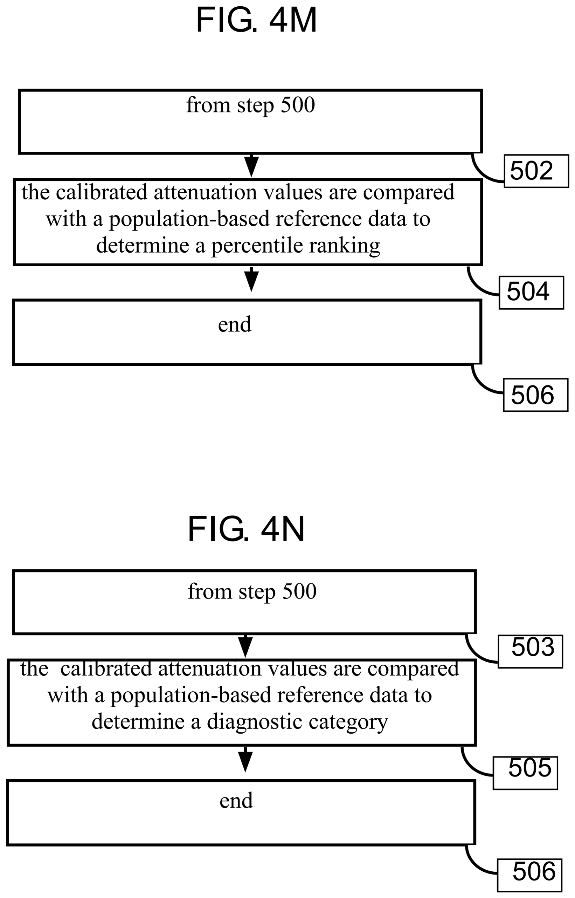

**FIG. 4B** depicts a flow chart showing a schematic representation of the method depicted in FIG. **4A****,** where in step 421 (after step 420), in step 423 the segmented vertebrae are labelled and then in step 431 the method returns to step 430 of the method depicted in **FIG. 4A****.** **FIG. 4C** depicts a flow chart showing a schematic representation of the method depicted in **FIG. 4A****,** where in step 421 (after step 420), in step 425 each segmented vertebra is checked to determine that they form a single connected vertebra and then in step 431 the method returns to step 430 of the method depicted in **FIG. 4A****.** **FIG. 4D** depicts a flow chart showing a schematic representation of the method depicted in **FIG. 4A****,** where in step 421 (after step 420), in step 427 each segmented vertebra is checked to determine that they are in the correct anatomical sequence and then in step 431 the method returns to step 430 of the method depicted in **FIG. 4A****.** **FIG. 4E** depicts a flow chart showing a schematic representation of the method depicted in **FIG. 4D****,** where in step 428 (after step 427), in step 420 consistency checks are performed on the one or more segmented vertebrae to ensure valid identification of the one or more segmented vertebrae and then in step 431 the method returns to step 430 of the method depicted in **FIG. 4A****.** **FIG. 4F** depicts a flow chart showing a schematic representation of the method depicted in **FIG. 4A****,** where in step 432 (after step 430), in step 433 the volume of the three-dimensional spherical Region of Interest is a predetermined percentage of a volume of the one or more segmented vertebrae and then in step 441 the method returns to step 440 of the method depicted in **FIG. 4A****.** **FIG. 4G** depicts a flow chart showing a schematic representation of the method depicted in **FIG. 4A****,** where the method is modified in step 452 (which follows step 450), and in step 454 where the position of the spherical ROI avoids the posterior central region of the one or more segmented vertebrae where a basivertebral foramen is located and then in step 461 the method returns to step 460 of the method depicted in **FIG. 4A****.** **FIG. 4H** depicts a flow chart showing a schematic representation of the method depicted in **FIG. 4A****,** where the method is modified in step 453 (which follows step 450), and in step 456 where the position targets a region of relatively low attenuation within the one or more segmented vertebrae and then in step 462 the method returns to step 460 of the method depicted in **FIG. 4A****.** **FIG. 4J** depicts a flow chart showing a schematic representation of the method depicted in **FIG. 4A****,** where the method is modified in step 463 (which follows step 460), and in step 465 where the plurality of voxel intensities is in Hounsfield Units and then in step 471 the method returns to step 470 of the method depicted in **FIG. 4A****.** **FIG. 4K** depicts a flow chart showing a schematic representation of the method depicted in **FIG. 4A****,** where the method is modified in step 472 (which follows step 470), and in step 474, where representative attenuation values are calculated by computing a median of the plurality of voxel intensities and then in step 481 the method returns to step 480 of the method depicted in **FIG. 4A****.** **FIG. 4L** depicts a flow chart showing a schematic representation of the method depicted in **FIG. 4A****,** where the method is modified in step 482 (which follows step 480), and in step 485, where the acquisition parameters are the model of the scanner or the X-ray tube voltage used to generate the 3DCTI, according to an embodiment of the invention and then in step 501 the method returns to step 500 of the method depicted in **FIG. 4A****.** **FIG. 4M** depicts a flow chart showing a schematic representation of the method depicted in **FIG. 4A****,** where the method is modified in step 502 (which follows step 500), and in step 504, where the calibrated attenuation values are compared with a population-based reference data stratified by one or both age and sex to determine a percentile ranking and then in step 506 the method ends. **FIG. 4N** depicts a flow chart showing a schematic representation of the method depicted in **FIG. 4A****,** where the method is modified in step 503 (which follows step 500), and in step 505 where the calibrated attenuation values are compared with a population-based reference data stratified by one or both age and sex to determine a diagnostic category and then in step 506 the method ends.

Overall, this invention provides a comprehensive solution for opportunistic bone health assessment using CT scans, addressing prior deficiencies and enabling widespread screening. By catching osteoporosis earlier, it can help initiate treatment sooner, ultimately aiming to reduce fracture incidence and improve patient outcomes.

Described above are methods and systems for implementing an AI based BMD assessment from CT scans. The foregoing description of embodiments of the methods, systems, and components of the present invention has been provided for the purposes of illustration and description. It is not intended to be exhaustive or to limit the invention to the precise forms disclosed. Many modifications and variations will be apparent to one of ordinary skill in the relevant arts. For example, steps performed in the embodiments of the invention disclosed can be performed in alternate orders, certain steps can be omitted, and additional steps can be added. The embodiments were chosen and described in order to best explain the principles of the invention and its practical application, thereby enabling others skilled in the art to understand the invention for various embodiments and with various modifications that are suited to the particular used contemplated. Other embodiments are possible and are covered by the invention. Such embodiments will be apparent to persons skilled in the relevant art(s) based on the teachings contained herein. The breadth and scope of the present invention should not be limited by any of the above-described exemplary embodiments, but should be defined only in accordance with the following claims and their equivalents.

### FURTHER EMBODIMENTS OF THE INVENTION

Overall, this invention provides a comprehensive solution for generating a RR, AR or IRR for a disease or malady. Determining AR can help monitoring / initiating treatment sooner, and ultimately aiming to reduce the disease or malady incidence and improve patient outcomes.

Described above are methods and systems for implementing an AI based RR, AR or IRR assessment from 3D image scans. The foregoing description of embodiments of the methods, systems, and components of the present invention has been provided for the purposes of illustration and description. It is not intended to be exhaustive or to limit the invention to the precise forms disclosed. Many modifications and variations will be apparent to one of ordinary skill in the relevant arts. For example, steps performed in the embodiments of the invention disclosed can be performed in alternate orders, certain steps can be omitted, and additional steps can be added. The embodiments were chosen and described in order to best explain the principles of the invention and its practical application, thereby enabling others skilled in the art to understand the invention for various embodiments and with various modifications that are suited to the particular used contemplated. Other embodiments are possible and are covered by the invention. Such embodiments will be apparent to persons skilled in the relevant art(s) based on the teachings contained herein. The breadth and scope of the present invention should not be limited by any of the above-described exemplary embodiments, but should be defined only in accordance with the claims and their equivalents.

Abbreviations used in the following examples and elsewhere herein are: 2D = two dimensional; 3D = three-Dimensional; CTI = Computer Tomography Image; AI = Artificial Intelligence; AIC = Artificial Intelligence Classifier; AR = Absolute Risk; AUC = Area under the Curve; BA = Brain Aneurysm; BF = Basivertebral Foramen; BMD = Bone Mineral Density; CBP = Cohort Baseline Prevalence; CD = Celiac Disease; CDF = Cumulative Distribution Function; CCDF = Complementary Cumulative Distribution Function; CMRI = Cardiac MRI; CNN = Convolutional Neural Network; CT = Computer Tomography; CTA = Computer Tomography Angiography; CTE = Computer Tomography Enterography; DBT = Digital Breast Tomosynthesis; DCE = Dynamic Contrast-Enhanced; DICOM = Digital Imaging and Communication in Medicine; DXA = Dual-energy X-ray Absorptiometry; HBP = Hepatobiliary phase; HU = Hounsfield Units; HUcalibrated = Calibrated Hounsfield Units; · HUmeasured = Measured Hounsfield Units; IBD = Inflammatory Bowel Disease; IRR = Instantaneous Risk Ratio; KNN = K-Nearest Neighbor; LV = Left Ventricular; MRI = Magnetic Resonance Imaging; MRE = Magnetic Resonance Enterography; MIP = Maximum Intensity Projection; MPR = Multi Planar Reformats; MRA= Magnetic Resonance Angiography; NN = Neural Networks; PACS = Picture Archiving and Communication System; PDF = Probability Density Function; PE = Pulmonary Embolism; PN = Peripheral Neuropathy; QCT = Quantitative CT; ROC curve = Receiver-Operating-Characteristic curve; ROI = Region of Interest; RR(S) = RR = Risk Ratio; RV = Rendered Volume; VDBTI = Volumetric Digital Breast Tomosynthesis Image; VI = Volumetric Image; VR = Volume Rendering; *S* = Continuous Confidence Score; SegV = Segmented Vertebrae; SROI = spherical ROI; SVM = Support Vector Machine; TP = true-positives; 3DIDS = three-Dimensional Image Data Set.

### FURTHER EMBODIMENTS OF THE INVENTION

Additional Examples S1 to S43 as follows:
Example S1. A method including (A) receiving (a) a plurality of X-ray images of a plurality of patients, and (b) a plurality of true positives corresponding to an incidence of breast cancer in the plurality of X-ray images, (B) generating a plurality of VDBTI from each of the plurality of X-ray images, (C) generating a plurality of scores for the incidence of breast cancer from the plurality of VDBTI using an AI classifier, where there is a correlation between each true positive of the plurality of true positives and a score from the plurality of scores, (D) converting the plurality of scores to a plurality of percentile values, (E) generating a smooth cumulative incidence curve using a first AIC based on the plurality of percentile values and the plurality of true positives, (F) calculating an Analytic derivative, where the Analytic derivative is a fit of a differentiable cumulative distribution function to the smooth cumulative incidence curve, (G) receiving one or both (c) a series of X-ray images of a breast of a patient adapted to generate a VDBTI and (d) the VDBTI of the patient from a user, (H) calculating a patient score for breast cancer from the series of X-ray images or the VDBTI, (I) computing a RR based on the patient score and the Analytic derivative.
Example S2. The method of Example S1, where, (i) the plurality of X-ray images of step (A)(a) are historical data, (ii) the series of X-ray images of step (G)(c) and the VDBTI of step (G)(d) are historical data, (iii) the differentiable cumulative distribution function is a mixture of at least two Beta cumulative-distribution functions, (iv) the differentiable cumulative distribution function is fitted using a non-parametric smoothing spline or a kernel estimator.
Example S3. The method of Example S1, further including (v) receiving a CBP for breast cancer, (J) calculating an AR that the patient suffers from breast cancer using the CBP and the RR, (vi) multiplying the Analytic derivative by a scaling factor corresponding to a number of score bins to obtain an IRR, (K) providing one or more of the RR, the AR and the IRR to the user.
Example S4. The method of Example S1, where, (vii) the smooth cumulative incidence curve and the RR generated therefrom is generated using a second AIC, (viii) comparing the RR generated using the first AIC with the RR generated using the second AIC, (ix) where converting the plurality of scores to the plurality of percentile values enables the second AIC to be directly compared with the first AIC.
Example S5. The method of Example S1, where the patient score is based at least in part on, (x) a thickening of a breast's skin, (xi) an asymmetry in a breast's density, (xii) an axillary lymphadenopathy of the patient.
Example S6. A method including (A) receiving (a) a first plurality of 3D data sets, each 3D data set of the first plurality of 3D sets comprising a second plurality of 2D images of an organ in a plurality of patients, and (b) a plurality of true positives for an incidence of a disease or a malady in the organ in the first plurality of 3D data sets, (B) generating a third plurality of 3D RVs from each of the first plurality of 3D data sets, (C) generating a plurality of scores correlating with the incidence of the disease or the malady in the organ from the third plurality of 3D RVs using an AI classifier, (D) converting the plurality of scores to a plurality of percentile values, (E) generating a smooth cumulative incidence curve using a first AIC based on the plurality of percentile values and the plurality of true positives, (F) calculating an Analytic derivative, where the Analytic derivative is a fit of a differentiable cumulative distribution function to the smooth cumulative incidence curve, (G) receiving one or both (c) a series of 2D images of the organ in a patient adapted to generate a 3D RV and (d) the 3D RV from a user, (H) calculating a patient score for the disease or the malady in the organ from the 3D RV, (I) computing a RR based on the patient score and the Analytic derivative.
Example S7. The method of Example S6, where, (i) the second plurality of 2D images of step (A)(a) are historical data, (ii) the series of 2D images of step (G)(c) and the 3D RV of step (G)(d) are historical data, (iii) the second plurality of 2D images are one or more of a plurality of x-ray images, a plurality of Computer Tomography Images and a plurality of Magnetic Resonance Images of patients, (iv) the differentiable cumulative distribution function is a mixture of at least two Beta cumulative-distribution functions, (v) the differentiable cumulative distribution function is fitted using a non-parametric smoothing spline or a kernel estimator, (vi) there is a correlation between each true positive of the plurality of true positives and a score from the plurality of scores.
Example S8. The method of Example S6, further including (vii) receiving a CBP for the disease or the malady, (J) calculating an AR that the patient suffers from the disease or the malady using the CBP and the RR, (viii) multiplying the Analytic derivative by a scaling factor corresponding to a number of score bins to obtain an IRR, (K) providing one or more of the RR, the AR and the IRR to the user.
Example S9. The method of Example S6, where, (ix) the smooth cumulative incidence curve and the RR generated therefrom is generated using a second AIC, (x) comparing the RR generated using the first AIC with the RR generated using the second AIC, (xi) where converting the plurality of scores to the plurality of percentile values enables the second AIC to be directly compared with the first AIC.
Example S10. A method including (A) receiving (a) a first plurality of 3D data sets from a user, each 3D data set of the first plurality of 3D sets comprising a second plurality of 2D images and (b) a plurality of true positives for an incidence of a disease or a malady from the first plurality of 3D data sets, (B) generating a third plurality of 3D RVs from each of the first plurality of 3D data sets, (C) generating a plurality of scores correlating with the incidence of the disease or the malady from the third plurality of 3D RVs using an AI classifier, where the plurality of scores are between 0 and 100, (D) converting the plurality of scores to a plurality of percentile values, (E) generating a smooth cumulative incidence curve using a first AIC based on the plurality of percentile values and the plurality of true positives, (F) calculating an Analytic derivative, where the Analytic derivative is a fit of a differentiable cumulative distribution function to the smooth cumulative incidence curve, (G) receiving one or both (c) a series of 2D images of a patient adapted to generate a 3D RV and (d) the 3D RV of the patient from the user, (H) calculating a patient score for the disease or the malady from the 3D RV, (I) computing a RR based on the patient score and the Analytic derivative.
Example S11. The method of Example S10, where, (i) the second plurality of 2D images of step (A)(a) are historical data, (ii) the series of 2D images of step (G)(c) and the 3D RV of step (G)(d) are historical data, (iii) the second plurality of 2D images generated are one or both CT Image scans and MR Imaging scans of patients suffering from the incidence of the disease or the malady, (vi) the differentiable cumulative distribution function is a mixture of at least two Beta cumulative-distribution functions, (v) the differentiable cumulative distribution function is fitted using a non-parametric smoothing spline or a kernel estimator, (vi) there is a correlation between each true positive of the plurality of true positives and a score from the plurality of scores.
Example S12. The method of Example S10, further including (vii) receiving a CBP for the disease or the malady, (J) calculating an AR that the patient suffers from the disease or the malady using the CBP and the RR, (vi) multiplying the Analytic derivative by a scaling factor corresponding to a number of score bins to obtain an IRR, (K) providing one or more of the RR, the AR and the IRR to the user.
Example S13. The method of Example S10, where, (ix) the smooth cumulative incidence curve and the RR generated therefrom is generated using a second AIC, (x) comparing the RR generated using the first AIC with the RR generated using the second AIC, (xi) where converting the plurality of scores to the plurality of percentile values enables the second AIC to be directly compared with the first AIC.
Example S14. A method including (A) receiving (a) a first plurality of 3D data sets from a user, each 3D data set of the first plurality of 3D sets comprising one or both a second plurality of 2D CT images of an organ and a third plurality of 2D MR images of the organ, and (b) a plurality of true positives for an incidence of a disease or a malady in the organ in the first plurality of 3D data sets, (B) generating a fourth plurality of 3D RVs from each of the first plurality of 3D data sets, (C) generating a plurality of scores for the incidence of the disease or the malady from the fourth plurality of 3D RVs using an AI classifier, where there is a correlation between each true positive of the plurality of true positives and a score from the plurality of scores, (D) converting the plurality of scores to a plurality of percentile values, (E) generating a smooth cumulative incidence curve using a first AIC based on the plurality of percentile values and the plurality of true positives, (F) calculating an Analytic derivative, where the Analytic derivative is a fit of a differentiable cumulative distribution function to the smooth cumulative incidence curve, (G) receiving one or both (c) a series of 2D CT images of the organ in a patient adapted to generate a 3D RV and (d) the 3D RV of the patient from the user, (H) calculating a patient score from the 3D RV, (I) computing a RR based on the patient score and the Analytic derivative.
Example S15. The method of Example S14, where, (i) the second plurality of 2D CT images of step (A)(a) are historical data, (ii) the series of 2D CT images of step (G)(c) and the 3D RV of step (G)(d) are historical data, (iii) the differentiable cumulative distribution function is a mixture of at least two Beta cumulative-distribution functions, (iv) the differentiable cumulative distribution function is fitted using a non-parametric smoothing spline or a kernel estimator.
Example S16. The method of Example S14, further including (v) receiving a CBP for the disease or the malady in the organ, (J) calculating an AR that the patient suffers from the disease or the malady in the organ using the CBP and the RR, (vi) multiplying the Analytic derivative by a scaling factor corresponding to a number of score bins to obtain an IRR, (K) providing one or more of the RR, the AR and the IRR to the user.
Example S17. The method of Example S14, where, (vii) the smooth cumulative incidence curve and the RR generated therefrom is generated using a second AIC, (viii) comparing the RR generated using the first AIC with the RR generated using the second AIC, (ix) where converting the plurality of scores to the plurality of percentile values enables the second AIC to be directly compared with the first AIC.
Example S18. The method of Example S14, where the patient score is based at least in part on a physiological change in the organ of the patient, where the physiological change is diagnostic of the disease or the malady.
Example S19. A method including (A) receiving (a) a first plurality of 3D data sets from a user, each 3D data set of the first plurality of 3D sets comprising a second plurality of 2D breast X-ray images and (b) a plurality of true positives for a re-occurrence of breast cancer from the first plurality of 3D data sets, (B) generating a third plurality of 3D RVs from each of the first plurality of 3D data sets, (C) generating a plurality of scores for the re-occurrence of breast cancer from the third plurality of 3D RVs using an AI classifier, where there is a correlation between each true positive of the plurality of true positives and a score from the plurality of scores, (D) converting the plurality of scores to a plurality of percentile values, (E) generating a smooth cumulative incidence curve using a first AIC based on the plurality of percentile values and the plurality of true positives, (F) calculating an Analytic derivative, where the Analytic derivative is a fit of a differentiable cumulative distribution function to the smooth cumulative incidence curve, (G) receiving one or both (c) a series of 2D X-ray images of a breast of a patient adapted to generate a VDBTI and (d) the VDBTI of the patient from the user, (H) calculating a patient score for the re-occurrence of breast cancer from the VDBTI, (I) computing a RR based on the patient score and the Analytic derivative.
Example S20. The method of Example S19, where, (i) one or both the second plurality of 2D breast X-ray images and the first plurality of 3D data sets are historical data, (ii) the series of 2D X-ray images and the VDBTI are historical data, (iii) the differentiable cumulative distribution function is a mixture of at least two Beta cumulative-distribution functions, (iv) the differentiable cumulative distribution function is fitted using a non-parametric smoothing spline or a kernel estimator.
Example S21. The method of Example S19, further including (v) receiving a CBP for recurrence of breast cancer, (J) calculating an AR that the patient suffers from recurrence of breast cancer using the CBP and the RR, (vi) multiplying the Analytic derivative by a scaling factor corresponding to a number of score bins to obtain an IRR, (K) providing one or more of the RR, the AR and the IRR to the user.
Example S22. The method of Example S19, where, (vii) the smooth cumulative incidence curve and the RR generated therefrom is generated using a second AIC, (viii) comparing the RR generated using the first AIC with the RR generated using the second AIC, (ix) where converting the plurality of scores to the plurality of percentile values enables the second AIC to be directly compared with the first AIC.
Example S23. The method of Example S19, where the patient score is based at least in part on, (x) a thickening of a breast's skin of the patient, (xi) an asymmetry in a breast's density of the patient, (xii) an axillary lymphadenopathy of the patient.
Example S24. A method including (A) receiving (a) a first plurality of 3D data sets from a user, each 3D data set of the first plurality of 3D sets comprising a second plurality of 2D MR images and (b) a plurality of true positives for a brain aneurysm from the first plurality of 3D data sets, (B) generating a third plurality of 3D RVs from each of the first plurality of 3D data sets, (C) generating a plurality of scores for an incidence of the brain aneurysm from the third plurality of 3D RVs using an AI classifier, where there is a correlation between each true positive of the plurality of true positives and a score from the plurality of scores, (D) converting the plurality of scores to a plurality of percentile values, (E) generating a smooth cumulative incidence curve using a first AIC based on the plurality of percentile values and the plurality of true positives, (F) calculating an Analytic derivative, where the Analytic derivative is a fit of a differentiable cumulative distribution function to the smooth cumulative incidence curve, (G) receiving one or both (c) a series of 2D images of a brain in a patient adapted to generate a 3D RV and (d) the 3D RV of the patient from the user, (H) calculating a patient score for the incidence of the brain aneurysm from the 3D RV, (I) computing a RR based on the patient score and the Analytic derivative.
Example S25. The method of Example S24, where, (i) the second plurality of 2D MR images of step (A)(a) are historical data, (ii) the series of 2D images of step (G)(c) and the 3D RV of step (G)(d) are historical data, (iii) the differentiable cumulative distribution function is a mixture of at least two Beta cumulative-distribution functions, (iv) the differentiable cumulative distribution function is fitted using a non-parametric smoothing spline or a kernel estimator.
Example S26. The method of Example S24, further including (v) receiving a CBP for the brain aneurysm, (J) calculating an AR that the patient suffers from the brain aneurysm using the CBP and the RR, (vi) multiplying the Analytic derivative by a scaling factor corresponding to a number of score bins to obtain an IRR, (K) providing one or more of the RR, the AR and the IRR to the user.
Example S27. The method of Example S24, where, (vii) the smooth cumulative incidence curve and the RR generated therefrom is generated using a second AIC, (viii) comparing the RR generated using the first AIC with the RR generated using the second AIC, (ix) where converting the plurality of scores to the plurality of percentile values enables the second AIC to be directly compared with the first AIC.
Example S28. The method of Example S24, where the images are generated using Dynamic Contrast-Enhanced imaging.
Example S29. A method including (A) receiving (a) a first plurality of 3D data sets from a user, each 3D data set of the first plurality of 3D sets comprising a second plurality of 2D CTE images and (b) a plurality of true positives for Crohn's disease from the first plurality of 3D data sets, (B) generating a third plurality of 3D RVs from each of the first plurality of 3D data sets, (C) generating a plurality of scores for an incidence of Crohn's disease from the third plurality of 3D RVs using an AI classifier, where there is a correlation between each true positive of the plurality of true positives and a score from the plurality of scores, (D) converting the plurality of scores to a plurality of percentile values, (E) generating a smooth cumulative incidence curve using a first AIC based on the plurality of percentile values and the plurality of true positives, (F) calculating an Analytic derivative, where the Analytic derivative is a fit of a differentiable cumulative distribution function to the smooth cumulative incidence curve, (G) receiving one or both (c) a series of 2D CTE images of a patient gut adapted to generate a 3D RV and (d) the 3D RV of a patient from the user, (H) calculating a patient score for the incidence of Crohn's disease from the 3D RV, (I) computing a RR based on the patient score and the Analytic derivative.
Example S30. The method of Example S29, where, (i) the second plurality of 2D CTE images of step (A)(a) are historical data, (ii) the series of 2D CTE images of step (G)(c) and the 3D RV of step (G)(d) are historical data, (iii) the differentiable cumulative distribution function is a mixture of at least two Beta cumulative-distribution functions, (iv) the differentiable cumulative distribution function is fitted using a non-parametric smoothing spline or a kernel estimator.
Example S31. The method of Example S29, further including (v) receiving a CBP for Crohn's disease, (J) calculating an AR that the patient suffers from Crohn's disease using the CBP and the RR, (vi) multiplying the Analytic derivative by a scaling factor corresponding to a number of score bins to obtain an IRR, (K) providing one or more of the RR, the AR and the IRR to the user.
Example S32. The method of Example S29, where, (vii) the smooth cumulative incidence curve and the RR generated therefrom is generated using a second AIC, (viii) comparing the RR generated using the first AIC with the RR generated using the second AIC, (ix) where converting the plurality of scores to the plurality of percentile values enables the second AIC to be directly compared with the first AIC.
Example S33. The method of Example S29, where the patient score is based in part on, (x) a first thickening of a patient's bowel wall, (xi) a second thickening of a patient's small intestine, (xii) presence of fistulas, abscesses, and / or intestinal blockages.
Example S34. A method including (A) receiving (a) a first plurality of 3D data sets from a user, each 3D data set of the first plurality of 3D sets comprising a second plurality of 2D MR images and (b) a plurality of true positives for an arrythmia from the first plurality of 3D data sets, (B) generating a third plurality of 3D RVs from each of the first plurality of 3D data sets, (C) generating a plurality of scores for an incidence of the arrythmia from the third plurality of 3D RVs using an AI classifier, where there is a correlation between each true positive of the plurality of true positives and a score from the plurality of scores, (D) converting the plurality of scores to a plurality of percentile values, (E) generating a smooth cumulative incidence curve using a first AIC based on the plurality of percentile values and the plurality of true positives, (F) calculating an Analytic derivative, where the Analytic derivative is a fit of a differentiable cumulative distribution function to the smooth cumulative incidence curve, (G) receiving one or both (c) a series of 2D MR images of a patient adapted to generate a 3D RV and (d) the 3D RV of the patient from the user, (H) calculating a patient score for the incidence of the arrythmia from the 3D RV, (I) computing a RR based on the patient score and the Analytic derivative.
Example S35. The method of Example S34, where, (i) the second plurality of 2D MR images of step (A)(a) are historical data, (ii) the series of 2D MR images of step (G)(c) and the 3D RV of step (G)(d) are historical data, (iii) the differentiable cumulative distribution function is a mixture of at least two Beta cumulative-distribution functions, (iv) the differentiable cumulative distribution function is fitted using a non-parametric smoothing spline or a kernel estimator.
Example S36. The method of Example S34, further including (v) receiving a CBP for the arrythmia, (J) calculating an AR that the patient suffers from the arrythmia using the CBP and the RR, (vi) multiplying the Analytic derivative by a scaling factor corresponding to a number of score bins to obtain an IRR, (K) providing one or more of the RR, the AR and the IRR to the user.
Example S37. The method of Example S34, where, (vii) the smooth cumulative incidence curve and the RR generated therefrom is generated using a second AIC, (viii) comparing the RR generated using the first AIC with the RR generated using the second AIC, (ix) where converting the plurality of scores to the plurality of percentile values enables the second AIC to be directly compared with the first AIC.
Example S38. The method of Example S34, where the patient score is based in part on, (x) an irregularity in a heart rhythm, (xi) a bradycardia, (xii) a tachycardia.
Example S39. A method including (A) receiving (a) a first plurality of 3D data sets, each 3D data set of the first plurality of 3D sets comprising a second plurality of 2D CT images and (b) a plurality of true positives for osteoporosis from the first plurality of 3D data sets, (B) generating a third plurality of 3D RVs from each of the first plurality of 3D data sets, (C) generating a plurality of scores for an incidence of osteoporosis from the third plurality of 3D RVs using an AI classifier, where there is a correlation between each true positive of the plurality of true positives and a HU score from the plurality of scores, (D) converting the plurality of scores to a plurality of percentile values, (E) generating a smooth cumulative incidence curve using a first AIC based on the plurality of percentile values and the plurality of true positives, (F) calculating an Analytic derivative, where the Analytic derivative is a fit of a differentiable cumulative distribution function to the smooth cumulative incidence curve, (G) receiving one or both (c) a series of 2D CT images of trabecular bone of vertebral bodies of a spine of a patient adapted to generate a 3D RV and (d) the 3D RV from a user, (H) calculating a patient HU score for the incidence of osteoporosis from the 3D RV, (I) computing a RR based on the patient HU score and the Analytic derivative.
Example S40. The method of Example S39, where, (i) the second plurality of 2D CT images of step (A)(a) are historical data, (ii) the series of 2D CT images of step (G)(c) and the 3D RV of step (G)(d) are historical data, (iii) the differentiable cumulative distribution function is a mixture of at least two Beta cumulative-distribution functions, (iv) the differentiable cumulative distribution function is fitted using a non-parametric smoothing spline or a kernel estimator.
Example S41. The method of Example S39, further including (v) receiving a CBP for osteoporosis, (J) calculating an AR that the patient suffers from osteoporosis using the CBP and the RR, (vi) multiplying the Analytic derivative by a scaling factor corresponding to a number of score bins to obtain an IRR, (K) providing one or more of the RR, the AR and the IRR to the user.
Example S42. The method of Example S39, where, (vii) the smooth cumulative incidence curve and the RR generated therefrom is generated using a second AIC, (viii) comparing the RR generated using the first AIC with the RR generated using the second AIC, (ix) where converting the plurality of scores to the plurality of percentile values enables the second AIC to be directly compared with the first AIC.
Example S43. The method of Example S39, where the patient HU score is based in part on, (x) a prior diagnosis of osteopenia, (xi) a diagnosis of degeneration of the spine, (xii) a DXA (Dual-energy X-ray Absorptiometry) scan T-score.

## Claims

1. A method comprising:
(A) receiving (a) a plurality of X-ray images of a plurality of patients, and (b) a plurality of true positives corresponding to an incidence of breast cancer in the plurality of X-ray images;
(B) generating a plurality of VDBTI (Volumetric Digital Breast Tomosynthesis Images) from each of the plurality of X-ray images;
(C) generating a plurality of scores for the incidence of breast cancer from the plurality of VDBTI using an AI classifier, where there is a correlation between each true positive of the plurality of true positives and a score from the plurality of scores;
(D) converting the plurality of scores to a plurality of percentile values;
(E) generating a smooth cumulative incidence curve using a first AIC (Artificial Intelligence Classifier) based on the plurality of percentile values and the plurality of true positives;
(F) calculating an Analytic derivative, where the Analytic derivative is a fit of a differentiable cumulative distribution function to the smooth cumulative incidence curve;
(G) receiving one or both (c) a series of X-ray images of a breast of a patient adapted to generate a VDBTI and (d) the VDBTI of the patient from a user;
(H) calculating a patient score for breast cancer from the series of X-ray images or the VDBTI; and
(I) computing a RR (Risk Ratio) based on the patient score and the Analytic derivative.

2. The method of claim 1, where:
(i) the plurality of X-ray images of step (A)(a) are historical data;
(ii) the series of X-ray images of step (G)(c) and the VDBTI of step (G)(d) are historical data;
(iii) the differentiable cumulative distribution function is a mixture of at least two Beta cumulative-distribution functions; and
(iv) the differentiable cumulative distribution function is fitted using a non-parametric smoothing spline or a kernel estimator;
the method further comprising:
(v) receiving a CBP (Cohort Baseline Prevalence) for breast cancer;
(J) calculating an AR (Absolute Risk) that the patient suffers from breast cancer using the CBP and the RR;
(vi) multiplying the Analytic derivative by a scaling factor corresponding to a number of score bins to obtain an IRR (Instantaneous Risk Ratio); and
(K) providing one or more of the RR, the AR and the IRR to the user.

3. The method of claim 1, where:
(vii) the smooth cumulative incidence curve and the RR generated therefrom is generated using a second AIC;
(viii) comparing the RR generated using the first AIC with the RR generated using the second AIC; and
(ix) where converting the plurality of scores to the plurality of percentile values enables the second AIC to be directly compared with the first AIC;
where optionally the patient score is based at least in part on:
(x) a thickening of a breast's skin;
(xi) an asymmetry in a breast's density; and
(xii) an axillary lymphadenopathy of the patient.

4. A non-transitory computer-readable medium storing instructions that, when executed by one or more processors, perform the method of any of claims 1-3.

5. A system comprising:
(A) an imaging workstation acquiring VDBTIs (Volumetric Digital Breast Tomosynthesis Images);
(B) an AI inference engine outputting continuous scores from VDBTIs;
(C) a calibration / analysis module implementing the method of any of claims 1-3; and
(D) a user interface displaying one or more of the RR, the AR, and the IRR.

6. The system of claim 5, where the user interface simultaneously displays one or more of the RR, the AR and the IRR for a given patient, visually aligned to a common scale.

7. A method comprising:
(A) receiving (a) a first plurality of 3D (three-Dimensional) data sets, each 3D data set of the first plurality of 3D sets comprising a second plurality of 2D (two-Dimensional) images of an organ in a plurality of patients, and (b) a plurality of true positives for an incidence of a disease or a malady in the organ in the first plurality of 3D data sets;
(B) generating a third plurality of 3D RVs (Rendered Volumes) from each of the first plurality of 3D data sets;
(C) generating a plurality of scores correlating with the incidence of the disease or the malady in the organ from the third plurality of 3D RVs using an AI classifier;
(D) converting the plurality of scores to a plurality of percentile values;
(E) generating a smooth cumulative incidence curve using a first AIC (Artificial Intelligence Classifier) based on the plurality of percentile values and the plurality of true positives;
(F) calculating an Analytic derivative, where the Analytic derivative is a fit of a differentiable cumulative distribution function to the smooth cumulative incidence curve;
(G) receiving one or both (c) a series of 2D images of the organ in a patient adapted to generate a 3D RV and (d) the 3D RV from a user;
(H) calculating a patient score for the disease or the malady in the organ from the 3D RV; and
(I) computing a RR (Risk Ratio) based on the patient score and the Analytic derivative.

8. The method of claim 7, where:
(i) the second plurality of 2D images of step (A)(a) are historical data;
(ii) the series of 2D images of step (G)(c) and the 3D RV of step (G)(d) are historical data;
(iii) the second plurality of 2D images are one or more of a plurality of x-ray images, a plurality of Computer Tomography Images and a plurality of Magnetic Resonance Images of patients;
(iv) the differentiable cumulative distribution function is a mixture of at least two Beta cumulative-distribution functions;
(v) the differentiable cumulative distribution function is fitted using a non-parametric smoothing spline or a kernel estimator; and
(vi) there is a correlation between each true positive of the plurality of true positives and a score from the plurality of scores.

9. The method of claim 7, further comprising:
(vii) receiving a CBP (Cohort Baseline Prevalence) for the disease or the malady;
(J) calculating an AR (Absolute Risk) that the patient suffers from the disease or the malady using the CBP and the RR;
(viii) multiplying the Analytic derivative by a scaling factor corresponding to a number of score bins to obtain an IRR (Instantaneous Risk Ratio); and
(K) providing one or more of the RR, the AR and the IRR to the user;
where optionally:
(ix) the smooth cumulative incidence curve and the RR generated therefrom is generated using a second AIC;
(x) comparing the RR generated using the first AIC with the RR generated using the second AIC; and
(xi) where converting the plurality of scores to the plurality of percentile values enables the second AIC to be directly compared with the first AIC.

10. A non-transitory computer-readable medium storing instructions that, when executed by one or more processors, perform the method of any of claims 7-9.

11. A system comprising:
(A) an imaging workstation acquiring 2D images;
(B) an AI inference engine outputting continuous scores;
(C) a calibration / analysis module implementing the method of any of claims 7-9; and
(D) a user interface displaying one or more of the RR, the AR and the IRR.

12. A method comprising:
(A) receiving (a) a first plurality of 3D (three-Dimensional) data sets, each 3D data set of the first plurality of 3D sets comprising a second plurality of 2D (two-Dimensional) CT (Computer Tomography) images and (b) a plurality of true positives for osteoporosis from the first plurality of 3D data sets;
(B) generating a third plurality of 3D RVs (Rendered Volumes) from each of the first plurality of 3D data sets;
(C) generating a plurality of scores for an incidence of osteoporosis from the third plurality of 3D RVs using an AI classifier, where there is a correlation between each true positive of the plurality of true positives and a HU (Hounsfield Units) score from the plurality of scores;
(D) converting the plurality of scores to a plurality of percentile values;
(E) generating a smooth cumulative incidence curve using a first AIC (Artificial Intelligence Classifier) based on the plurality of percentile values and the plurality of true positives;
(F) calculating an Analytic derivative, where the Analytic derivative is a fit of a differentiable cumulative distribution function to the smooth cumulative incidence curve;
(G) receiving one or both (c) a series of 2D CT images of trabecular bone of vertebral bodies of a spine of a patient adapted to generate a 3D RV and (d) the 3D RV from a user;
(H) calculating a patient HU score for the incidence of osteoporosis from the 3D RV; and
(I) computing a RR (Risk Ratio) based on the patient HU score and the Analytic derivative.

13. The method of claim 12, where:
(i) the second plurality of 2D CT images of step (A)(a) are historical data;
(ii) the series of 2D CT images of step (G)(c) and the 3D RV of step (G)(d) are historical data;
(iii) the differentiable cumulative distribution function is a mixture of at least two Beta cumulative-distribution functions; and
(iv) the differentiable cumulative distribution function is fitted using a non-parametric smoothing spline or a kernel estimator;
the method further optionally comprising:
(v) receiving a CBP (Cohort Baseline Prevalence) for osteoporosis;
(J) calculating an AR (Absolute Risk) that the patient suffers from osteoporosis using the CBP and the RR;
(vi) multiplying the Analytic derivative by a scaling factor corresponding to a number of score bins to obtain an IRR (Instantaneous Risk Ratio); and
(K) providing one or more of the RR, the AR and the IRR to the user.

14. The method of claim 12, where:
(vii) the smooth cumulative incidence curve and the RR generated therefrom is generated using a second AIC;
(viii) comparing the RR generated using the first AIC with the RR generated using the second AIC; and
(ix) where converting the plurality of scores to the plurality of percentile values enables the second AIC to be directly compared with the first AIC;
where optionally the patient HU score is based in part on:
(x) a prior diagnosis of osteopenia;
(xi) a diagnosis of degeneration of the spine; and
(xii) a DXA (Dual-energy X-ray Absorptiometry) scan T-score.

15. A non-transitory computer-readable medium storing instructions that, when executed by one or more processors, perform the method of any of claims 12-14.
